# EUROPÄISCHE PATENTSCHRIFT

(11) **EP 4 150 108 B1**
(45) Veröffentlichungstag und Bekanntmachung des Hinweises auf die Patenterteilung: **29.01.2025**
(21) Anmeldenummer: 21721940.1
(22) Anmeldetag: 30.04.2021
(51) Int. Cl.: C12Q 1/68, C12Q 1/6874

(54) **FRÜHERKENNUNG VON KRANKHEITSERREGERN BEI PFLANZEN**
EARLY RECOGNITION OF PATHOGENS IN PLANTS
DÉTECTION PRÉCOCE DES AGENTS PATHOGÈNES CHEZ LES PLANTES

(30) Priorität: 13.05.2020 EP 20174320; 03.07.2020 EP 20183910
(43) Veröffentlichungstag der Anmeldung: 22.03.2023
(73) Patentinhaber: Bayer Aktiengesellschaft, 51373 Leverkusen (DE)
(72) Erfinder: BUER, Benjamin, 50829 Köln (DE); AL DILAIMI-WENDLER, Arwa, 51375 Leverkusen (DE); MEHL, Andreas, 40670 Meerbusch (DE); KLEEMANN, Jochen, 50170 Kerpen (DE)
(74) Vertreter: BIP Patents
(86) Internationale Anmeldenummer: PCT/EP2021/061366
(87) Internationale Veröffentlichungsnummer: WO 2021/228578

(56) Entgegenhaltungen:
- EP-A1- 3 216 873
- WO-A1-2019/149626
- K. SCHMIDT ET AL: "Identification of bacterial pathogens and antimicrobial resistance directly from clinical urines by nanopore-based metagenomic sequencing", JOURNAL OF ANTIMICROBIAL CHEMOTHERAPY., vol. 72, no. 1, 25 September 2016 (2016-09-25), GB, pages 104 - 114, XP055443758, ISSN: 0305-7453, DOI: 10.1093/jac/dkw397
- MINHDUC CAO ET AL: "Streaming algorithms for identification of pathogens and antibiotic resistance potential from real-time MinION sequencing", GIGASCIENCE, BIOMED CENTRAL LTD, LONDON, UK, vol. 5, no. 1, 26 July 2016 (2016-07-26), pages 1 - 16, XP021269358, DOI: 10.1186/S13742-016-0137-2

## Beschreibung

Die vorliegende Erfindung befasst sich mit der Früherkennung von Krankheitserregern in, an oder auf Pflanzen oder Pflanzenteilen, sowie der Identifizierung von Anzeichen auf Resistenzen dieser Krankheitserreger gegenüber chemischen oder biologischen Bekämpfungsmitteln. Gegenstände der vorliegenden Erfindung sind ein System, ein Verfahren und ein computerlesbares Medium zur frühen Erkennung der Anwesenheit von Krankheitserregern in, an oder auf Pflanzen oder Pflanzenteilen mit Hilfe von Sequenzanalysen und der Identifizierung von Resistenzanzeichen.

Die frühzeitige Erkennung eines Befalls einer Pflanze mit einem Krankheitserreger ist sehr wichtig. Je früher ein Befall erkannt wird, desto eher können geeignete Maßnahmen ergriffen werden, um einen Ausbruch der Krankheit und/oder eine Ausbreitung zu verhindern und/oder Schaden von den Pflanzen abzuwenden.

WO2017/194276 beschreibt ein Verfahren zur Erkennung von Pflanzenkrankheiten, das auf der Analyse von Bildern eines Pflanzenteils basiert. Eine Pflanze kann jedoch von einer Population von Krankheitserregern befallen sein, ohne dass bereits sichtbare Symptome auftreten. Ferner kann der Resistenzstatus der Population von Krankheitserregern mittels einer derartigen Bildanalyse nicht ermittelt werden.

US20120088696A1 offenbart eine mikroelektrochemische Multiplex-PCR-Plattform, die zur Amplifikation, Untersuchung und Quantifizierung von Zielnukleotiden in Echtzeit verwendet werden kann und beispielsweise zum schnellen Nachweis von Pflanzenkrankheiten verwendet werden kann. Die Plattform muss jedoch im Einzelfall jeweils speziell für die Erkennung einer spezifischen Krankheit angepasst werden und sie ist vergleichsweise aufwändig in der Anwendung.

Vorteilhaft wäre es, wenn nicht nur Krankheitserreger frühzeitig erkannt, sondern in einem frühen Status auch bereits Resistenzen bei der Population von Krankheitserregern gegenüber einem Bekämpfungsmittel ermittelt werden könnten.

WO2019149626A1 befasst sich mit der Identifizierung von Resistenzmarkern im Feld.

Die vorliegende Erfindung stellt eine Ergänzung der in WO2019149626A1 beschriebenen Lösung dar. Die vorliegende Erfindung kombiniert die Ermittlung von Resistenzen mit einer frühzeitigen Erkennung von Krankheitserregern bei Pflanzen. Die in dieser Beschreibung vorgestellten Lösungen sind dabei effizient, anpassungsfähig und können weitere Informationen in die Früherkennung von Krankheitserregern und Resistenzen einbeziehen.

Ein erster Gegenstand der vorliegenden Erfindung ist ein Verfahren umfassend die Schritte:
- Sammeln einer Probe von einer Pflanze oder von einem Medium, in dem die Pflanze wächst,
- Identifizieren eines Pathogens in der Probe,
- Identifizieren von Anzeichen auf eine Resistenz des Pathogens gegenüber einem Bekämpfungsmittel,
   - wobei das Identifizieren des Pathogens und das Identifizieren der Anzeichen auf eine Resistenz mittels Sequenzieren von einzelnen Nukleinsäuren oder einzelnen Peptiden in der Probe erfolgt, wobei beim Sequenzieren der einzelnen Nukleinsäuren oder einzelnen Peptiden Sequenzen von Nukleotiden oder Sequenzen von Aminosäuren ermittelt werden, wobei die Sequenzen von Nukleotiden oder Sequenzen von Aminosäuren mit Referenzsequenzen verglichen werden, wobei das Sequenzieren in zwei Phasen erfolgt, einer ersten Phase und einer zweiten Phase,
   - wobei in der ersten Phase während des Sequenzierens einer jeden Nukleinsäure oder eines jeden Peptids die jeweils ermittelte Sequenz mit mindestens einer Pathogen-Sequenz verglichen wird, und
      ▪ für den Fall, dass die ermittelte Sequenz nicht mit der mindestens einen Pathogen-Sequenz übereinstimmt, die weitere Sequenzierung dieser Nukleinsäure oder dieses Peptids abgebrochen wird,
      ▪ für den Fall, dass die ermittelte Sequenz mit einer Pathogen-Sequenz übereinstimmt, ein Pathogen ermittelt wird, dass die Pathogen-Sequenz aufweist, und anhand des ermittelten Pathogens mindestens ein Resistenzmarker ermittelt wird, und in die zweite Phase gewechselt wird,
   - wobei in der zweiten Phase Sequenzen der Nukleinsäuren oder Peptide in der Probe mit dem mindestens einen ermittelten Resistenzmarker und/oder mit einer resistenzfreien Sequenz verglichen werden.

Ein weiterer Gegenstand der vorliegenden Erfindung ist ein System umfassend
- eine Sequenziereinheit,
- eine Steuer- und Berechnungseinheit und
- mindestens einen Datenspeicher, in dem für mindestens ein Pathogen mindestens eine Pathogen-Sequenz und mindestens ein Resistenzmarker gespeichert sind,

wobei die Sequenziereinheit konfiguriert ist, Nukleinsäuren oder Peptide in einer Probe von einer Pflanze oder von einem Medium, in dem die Pflanze wächst, zu sequenzieren, und dabei Sequenzen von Nukleotiden oder Aminosäuren in der Probe zu ermitteln,
wobei die Sequenziereinheit konfiguriert ist, während der Ermittlung der Sequenzen, die jeweils ermittelten Sequenzen an die Steuer- und Berechnungseinheit zu übermitteln,
wobei die Steuer- und Berechnungseinheit konfiguriert ist, in einer ersten Phase jede übermittelte Sequenz von einer Nukleinsäure oder einem Peptid mit mindestens einer Pathogen-Sequenz zu vergleichen, und
   - für den Fall, dass eine übermittelte Sequenz nicht mit der mindestens einen Pathogen-Sequenz übereinstimmt, die Sequenziereinheit zu veranlassen, die weitere Sequenzierung der jeweiligen Nukleinsäure oder des jeweiligen Peptids abzubrechen,
   - für den Fall, dass eine übermittelte Sequenz mit einer Pathogen-Sequenz übereinstimmt, ein Pathogen zu ermitteln, das die Pathogen-Sequenz aufweist, und anhand des ermittelten Pathogens mindestens einen Resistenzmarker zu ermitteln, und in eine zweite Phase zu wechseln,
wobei die Steuer- und Berechnungseinheit konfiguriert ist, in der zweiten Phase Sequenzen der Nukleinsäuren oder Peptide in der Probe mit dem mindestens einen ermittelten Resistenzmarker und/oder einer resistenzfreien Sequenz zu vergleichen.

Ein weiterer Gegenstand der vorliegenden Erfindung ist ein computerlesbares (Speicher-)Medium umfassend Befehle, die bei einer Ausführung durch einen Computer diesen veranlassen, folgende Schritte auszuführen:
- für eine Vielzahl von Nukleinsäuren oder Peptiden: Empfangen einer wachsenden Abfolge von Nukleotiden oder Aminosäuren in der jeweiligen Nukleinsäure oder dem jeweiligen Peptid von einer Sequenziereinheit,
- in einer ersten Phase während des Empfangens der wachsenden Abfolge: Prüfen, ob die Abfolge von Nukleotiden oder Aminosäuren ein- oder mehrfach in mindestens einer Pathogen-Sequenz enthalten ist,
- für den Fall, dass die Abfolge in keiner Pathogen-Sequenz enthalten ist: Übermitteln eines Signals an die Sequenziereinheit zum Abbruch der weiteren Sequenzierung der Nukleinsäure oder des Peptids,
- für den Fall, dass die Abfolge mit einer Pathogen-Sequenz übereinstimmt: Ermitteln eines Pathogens, das die Pathogen-Sequenz aufweist, und Ermitteln mindestens eines Referenzmarkers, und Initiieren einer zweiten Phase,
- in der zweiten Phase: Vergleichen der Abfolgen von Nukleotiden oder Aminosäuren mit dem mindestens einen Referenzmarker und/oder einer resistenzfreien Sequenz.

Ein weiterer Gegenstand der vorliegenden Erfindung ist ein Computerprogramm umfassend Befehle, die bei der Ausführung des Computerprogramms durch einen Computer den Computer veranlassen, folgende Schritte auszuführen:
- für eine Vielzahl von Nukleinsäuren oder Peptiden: Empfangen einer wachsenden Abfolge von Nukleotiden oder Aminosäuren in der jeweiligen Nukleinsäure oder dem jeweiligen Peptid von einer Sequenziereinheit,
- in einer ersten Phase während des Empfangens der wachsenden Abfolge: Prüfen, ob die Abfolge von Nukleotiden oder Aminosäuren ein- oder mehrfach in mindestens einer Pathogen-Sequenz enthalten ist,
- für den Fall, dass die Abfolge in keiner Pathogen-Sequenz enthalten ist: Übermitteln eines Signals an die Sequenziereinheit zum Abbruch der weiteren Sequenzierung der Nukleinsäure oder des Peptids,
- für den Fall, dass die Abfolge mit einer Pathogen-Sequenz übereinstimmt: Ermitteln eines Pathogens, das die Pathogen-Sequenz aufweist, und Ermitteln mindestens eines Referenzmarkers, und Initiieren einer zweiten Phase,
- in der zweiten Phase: Vergleichen der Abfolgen von Nukleotiden oder Aminosäuren mit dem mindestens einen Referenzmarker und/oder einer resistenzfreien Sequenz.

Bevorzugte Ausführungsformen der vorliegenden Erfindung finden sich in den abhängigen Patentansprüchen, der vorliegenden Beschreibung und in den Zeichnungen.

Die Erfindung wird nachstehend näher erläutert, ohne zwischen den Erfindungsgegenständen (Verfahren, System, computerlesbares Medium, Computerprogramm) zu unterscheiden. Die nachfolgenden Erläuterungen sollen vielmehr für alle Erfindungsgegenstände in analoger Weise gelten, unabhängig davon, in welchem Kontext (Verfahren, System, computerlesbares Medium, Computerprogramm) sie erfolgen.

Wenn in der vorliegenden Beschreibung oder in den Patentansprüchen Schritte in einer Reihenfolge genannt werden, bedeutet dies nicht zwingend, dass die Erfindung auf die genannte Reihenfolge beschränkt ist. Vielmehr ist es denkbar, dass die Schritte auch in einer anderen Reihenfolge oder auch parallel zueinander ausgeführt werden können; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar.

Die vorliegende Erfindung stellt Mittel bereit, die es einem Nutzer erlauben, frühzeitig einen Befall einer Pflanze mit Krankheitserregern und Anzeichen auf eine Resistenz der Krankheiterreger gegenüber einem Bekämpfungsmittel zu erkennen.

Unter dem Begriff "Krankheitserreger" werden Mikroorganismen oder subzelluläre Erreger verstanden, die in anderen Organismen gesundheitsschädigende Abläufe verursachen. Krankheitserreger können z.B. Algen, Bakterien, Parasiten, Pilze, Prionen, Protisten, Viren oder Viroide sein. In dieser Beschreibung wird der Begriff "Pathogen" synonym zum Begriff Krankheitserreger verwendet. Vorzugsweise befasst sich die vorliegende Erfindung mit der Früherkennung von pflanzenschädigenden Pilzen.

Unter dem Begriff "Bekämpfungsmittel" wird ein Mittel verstanden, mit dem Krankheitserreger wirksam bekämpft und/oder ihre Ausbreitung verhindert werden kann. Beispiele für Bekämpfungsmittel sind Fungizide, Insektizide und Herbizide. Ein Bekämpfungsmittel umfasst üblicherweise einen oder mehrere Wirkstoffe. "Wirkstoffe" sind chemische oder biologische Substanzen, die in einem Krankheitserreger eine spezifische Wirkung haben bzw. eine spezifische Reaktion hervorrufen.

Unter dem Begriff "Resistenz" wird eine Eigenschaft einzelner Krankheitserreger einer Art verstanden, die sich darin zeigt, dass diese Individuen eine Behandlung mit einem Bekämpfungsmittel, mit dem sich die Art normalerweise bekämpfen lässt, überstehen und ihren Entwicklungszyklus normal abschließen bzw. sich weiter verbreiten können.

Unter dem Begriff "Nutzer" wird ein Mensch verstanden, der die Erfindung ausführt; insbesondere ist der Nutzer ein Mensch, der das erfindungsgemäße System verwendet und/oder einen Computer nutzt, auf dem das erfindungsgemäße Computerprogramm installiert ist, um die Erfindung auszuführen.

Man kann zwei Typen von Resistenzen unterscheiden; die wirkortspezifische Resistenz, auch Target-Site-Resistenz genannt, und die metabolische Resistenz, auch Non-Target-Site-Resistenz genannt.

Eine wirkortspezifische Resistenz wird üblicherweise durch eine Mutation bei einem einzelnen Gen verursacht, das mit dem Wirkort (Target) eines Bekämpfungsmittels in dem Pathogen in Zusammenhang steht. Die Mutation kann zum Beispiel eine stärkere Expression des Zielproteins bewirken oder eine Veränderung der Stelle, an die der Wirkstoff des Bekämpfungsmittels bindet. Für diese Art der Resistenz gibt es molekularbiologische Tests.

Eine Resistenz ohne direkten Zusammenhang mit dem Wirkort (Non-Target-Site-Resistenz) ist auf alle anderen Mechanismen zurückzuführen, wie zum Beispiel eine verlangsamte Aufnahme des Bekämpfungsmittels durch den Krankheitserreger.

Mit der vorliegenden Erfindung können insbesondere Target-Site-Resistenzen frühzeitig erkannt werden.

Die Krankheitserreger/Pathogene, die im Fokus der vorliegenden Erfindung stehen, befallen vor allem Pflanzen, vorzugsweise Kulturpflanzen. Unter dem Begriff "Kulturpflanze" wird eine Pflanze verstanden, die durch das Eingreifen der Menschen zielgerichtet als Nutzpflanze angebaut wird. Teile der angebauten Kulturpflanze können zum menschlichen und/oder tierischen Verzehr geeignet sein.

Es ist grundsätzlich denkbar, die Lehre der vorliegenden Erfindung auch auf Tiere, insbesondere Nutztiere zu übertragen. Ein weiterer Gegenstand der vorliegenden Erfindung ist daher die Anwendung der in dieser Beschreibung aufgeführten Lehren auf Tiere, insbesondere Nutztiere.

In einem ersten Schritt wird mindestens eine Probe gesammelt. Der Begriff "Sammeln" ist in keiner Weise limitierend zu verstehen. Ein Beispiel eines synonymen Begriffs ist der Begriff "Probennahme".

Die Probe wird von der Pflanze oder aus dem Medium, in dem die Pflanze wächst, genommen. Der Begriff "Medium" soll neben dem Erdboden auch jedes andere Substrat umfassen, das im Kontakt mit der Pflanze steht und das der Pflanze Halt gibt und/oder sie mit Nährstoffen versorgt. Bei Wasserpflanzen kann auch eine Probe aus dem die Wasserpflanze umgebenden Wasser genommen werden.

Vorzugsweise wird als Probe ein Teil der Pflanze gesammelt, beispielsweise ein Blatt, eine Blüte, eine Sprossachse, eine Wurzel, Pollen, Rinde und/oder ein Teil der genannten Bestandteile und/oder mehrere der genannten Bestandteile und/oder andere Bestandteile. Auch von "Ausscheidungen" wie Blütennektar oder Harz kann eine Probe genommen werden. Vorzugsweise werden verschiedene Gewebearten der Pflanze zur Abdeckung verschiedenster Krankheitserreger entnommen und kombiniert. Vorzugsweise befindet sich die Pflanze zum Zeitpunkt der Probennahme in einem frühen Entwicklungsstadium.

Das Entwicklungsstadium einer Pflanze kann beispielsweise in Form des so genannten BBCH-Codes angegeben werden. Die Abkürzung BBCH steht offiziell für die Biologische Bundesanstalt, das Bundessortenamt und die CHemische Industrie. Die erweiterte BBCH-Skala zur einheitlichen Kodierung der phänologischen Entwicklungsstadien mono- und dikotyler Pflanzen ist eine Gemeinschaftsarbeit der Biologischen Bundesanstalt für Land- und Forstwirtschaft (BBA), des Bundessortenamtes (BSA), des Industrieverbandes Agrar (IVA) und des Instituts für Gemüse u. Zierpflanzenbau Großbeeren/Erfurt (siehe z.B. https://www.julius-kuehn.de/publikationsreihen-desjki/bbch-skalal).

Vorzugsweise befindet sich die Pflanze zum Zeitpunkt der Probennahme im Makrostadium 1 (Blattentwicklung (Hauptsproß)) oder Makrostadium 2 (Bildung von Seitensprossen) oder Makrostadium 3 (Längen- bzw. Rosettenwachstum des Hauptsprosses/Triebentwicklung/Schossen (Hauptrieb)) oder Makrostadium 4 (Entwicklung vegetativer Pflanzenteile bzw. vegetativer Vermehrungsorgane / Ähren- bzw. Rispenschwellen) oder Makrostadium 5 (Erscheinen der Blütenanlage) oder Makrostadium 6 (Blüte) der BBCH-Skala. Es wurde gefunden, dass Pilze und/oder Pilzsporen bereits in diesen frühen Stadien in, an oder auf einer Pflanze nachweisbar sind, ohne dass die Pflanze jedoch schon sichtbare Krankheitssymptome aufweisen muss. Eine Erkennung zu einem solchen frühen Zeitpunkt erlaubt eine frühzeitige Behandlung der Pflanze, um einen Ausbruch einer Krankheit und/oder die Vermehrung und/oder Ausbreitung der Krankheitserreger und/oder eine Übertragung von Krankheitserregern auf benachbarte Pflanzen zu unterbinden bzw. das Risiko hierfür zu reduzieren.

Die Probennahme kann automatisiert erfolgen. "Automatisiert" bedeutet, dass die Probennahme ohne ein Zutun eines Menschen durch eine Maschine oder durch mehrere Maschinen erfolgt. Eine solche Maschine oder Vorrichtung zur automatisierten Probennahme wird in dieser Beschreibung auch als "Probennahmeeinheit" bezeichnet.

Die Probennahme kann mit Hilfe einer mobilen Vorrichtung oder durch eine mobile Vorrichtung erfolgen, die sich zum Beispiel in einem Feld für Kulturpflanzen und/oder über das Feld bewegt oder bewegt wird. Denkbar ist z.B. die Verwendung einer (vorzugsweise unbemannten) Landmaschine und/oder eines (vorzugsweise unbemannten) Luftfahrzeugs (z.B. einer Drohne) und/oder eines Roboters. In einem Gewächshaus bietet sich die Verwendung eines Roboters für eine automatisierte Probennahme an.

Die Probennahme kann aber auch durch eine oder mehrere Vorrichtungen erfolgen, die stationär an einem Ort aufgebaut sind.

Denkbar ist ferner, dass ein Nutzer (Mensch) selbst eine Probennahme durchführt.

Die Probe wird einer Sequenziereinheit zugeführt. Die Sequenziereinheit ist konfiguriert, die Sequenzen von Nukleinsäuren oder Peptiden, die sich in der Probe befinden, zu bestimmen.

"Nukleinsäuren" sind aus einzelnen Bausteinen, den Nukleotiden, aufgebaute Makromoleküle, die bei Organismen und subzellulären Agentien (wie beispielsweise Viren) die genetische Information enthalten. Nukleinsäuren kommen in der Natur in zwei Formen vor: Desoxyribonukleinsäure (DNS) und Ribonukleinsäure (RNS). Die DNS ist das Erbmaterial aller Lebewesen, von einzelligen Bakterien bis zu mehrzelligen Säugetieren. Einige Viren verwenden RNS statt DNS als ihre Erbsubstanz. Vorzugsweise werden DNS-Stränge, die sich in einer Probe befinden, sequenziert.

Ein "Peptid" ist eine organische Verbindung, die Peptidbindungen zwischen Aminosäuren enthält. Lange Peptidketten werden auch als Proteine bezeichnet. Proteine finden sich in jeder Zelle und dienen ihr als molekulare "Werkzeuge" und erfüllen je nach der besonderen Struktur unterschiedliche Aufgaben, indem sie beispielsweise Zellbewegungen ermöglichen, Metabolite transportieren, Ionen pumpen, chemische Reaktionen katalysieren oder Signalstoffe erkennen können. In dieser Beschreibung werden die Begriffe "Peptid" und "Protein" synonym verwendet.

Unter dem Begriff "Sequenzierung/Sequenzieren" wird die Bestimmung der Nukleotid-Abfolge in einer Nukleinsäure bzw. die Bestimmung der Aminosäuren-Abfolge in einem Peptid (Protein) verstanden. Die Sequenz ist dementsprechend die Nukleotid-Abfolge bzw. die Aminosäuren-Abfolge. Die Begriffe "Sequenz" und "Abfolge" werden in dieser Beschreibung synonym verwendet.

Es ist denkbar, dass die Probe aufgearbeitet werden muss, bevor sie der Sequenzierung zugeführt werden kann. Bei einer solchen Aufarbeitung können Zellen in der Probe aufgeschlossen werden, zum Beispiel, um die DNS-Moleküle freizusetzen, sie anschließend zu extrahieren und ggf. zu fragmentieren. Die entsprechenden Maßnahmen zur Aufarbeitung sind im Stand der Technik beschrieben (siehe z.B. R. P. Schaudies (Ed.): Biological Identification, Woodhead Publishing Series in Electronic and Optical Materials: Number 59, Elsevier 2014, ISBN 978-0-85709-501-5; Jianping Xu: Next-generation Sequencing, Caister Academic Press 2014, ISBN 978-1-908230-33-1; Vijai Bhadauria: Next-generation Sequencing and Bioinformatics for Plant Science, Caister Academic Press 2017, ISBN 978-1-910190-65-4). Zur Probenaufbereitung kann eine Probenaufbereitungseinheit verwendet werden, die ein Bestandteil der Probennahmeeinheit und/oder der Sequenziereinheit oder eine separate Einheit sein kann.

Bei der Probenaufbereitung kann eine Amplifizierung von spezifischen Regionen der vorhandenen Nukleinsäuren erfolgen; eine Amplifizierung ist jedoch nicht unbedingt erforderlich. Eine Amplifizierung kann bei einer solchen Region sinnvoll sein, die für eine Spezies charakteristisch ist, bzw. anhand der eine Spezies eindeutig und/oder sicher identifiziert werden kann. Als Beispiel einer solchen Region sei die ITS-Region genannt.

Die ITS-Region (ITS: *Internal Transcribed Spacer)* ist ein bei phylogenetischen Untersuchungen häufig verwendete Nukleotid-Sequenz. Bei der ITS-Region handelt es sich um eine intergenische, nichtcodierende Region mit hoch variabler Sequenz; sie eignet sich deswegen besonders für die Bestimmung von Verwandtschaftsbeziehungen auf den unteren taxonomischen Niveaus der Arten, Gattungen und Familien. Eine Sequenzanalyse dieser Region kann insbesondere für die Gattungs- und Speziesidentifizierung von Pilzen genutzt werden (siehe z.B. T.M. Pryce et al.: Rapid identification of fungi by sequencing the ITS l and ITS2 regions using an automated capillary electrophoresis system; Medical Mycology October 2003, 4 1, 369-381; R. H. Nilsson et al.: A Comprehensive, Automatically Updated Fungal ITS Sequence Dataset for Reference-Based Chimera Control in Environmental Sequencing Efforts, Microbes Environ. 2015 Jun; 30(2): 145-150; A.J. Buehler et al.: Internal transcribed spacer (ITS) sequencing reveals considerable fungal diversity in dairy products, Dairy Sci. 2017; 100(11): 8814-8825; C. L. Schoch et al.: Nuclear ribosomal internal transcribed spacer (ITS) region as a universal DNA barcode marker for Fungi, PNAS April 17, 2012 109 (16) 6241-6246). Durch Vergleich der ermittelten ITS-Sequenz und der 5,8S rRNS-Gensequenz eines zu identifizierenden Pilzstammes werden Sequenzähnlichkeiten bestimmt, die als Grundlage für eine Zuordnung zu einer taxonomischen Gruppe dienen. Die Nukleotidsequenz der ITS-Region beträgt etwa 500-600 Basenpaare. Neben hoch konservierten Bereichen, in denen die Sequenz für alle Pilze identisch ist, kommen auch variable Bereiche vor. Diese variablen Bereiche können als Signaturen für eine Spezies, Gattung oder Gruppe von Pilzen charakteristisch sein. Somit ermöglicht die Analyse dieser Teilsequenzen eine taxonomische Zuordnung von Pilzstämmen.

Da die ITS-Region mehrfach als Genfamilie mit jeweils mehreren hundert- bis tausendfach tandemartig wiederholten Kopien auftritt, ist sie einem einzelnen Kerngen gegenüber stöchiometrisch weit überrepräsentiert und damit für molekularphylogenetische Untersuchungen leicht zugänglich. Ein weiterer Vorteil der Verwendung der ITS-Region für molekularphylogenetische Untersuchungen ergibt sich durch ihre Lage. Die ITS-Region liegt im rRNS-Gencluster. Sie besteht aus zwei Sequenzabschnitten, dem *Internal Transcribed Spacer 1* (ITS-1) und dem *Internal Transcribed Spacer* 2 (ITS-2), die zwischen den hoch konservierten rDNS-Genen 26S, 5,8S und 18S liegen. Durch diese Lage können rDNS-Sequenzabschnitte der hoch konservierten rDNS-Gene als Primer-Sequenzen genutzt werden. Eine Amplifizierung der ITS-1- und ITS-2-Sequenzen mittels PCR ist somit leicht möglich.

Die Sequenzierung von in der Probe vorhandenen Nukleinsäuren oder Peptiden wird durch die Sequenziereinheit vorgenommen. Vorzugsweise erfolgt die Sequenzierung mittels Nanotechnologiebasierten Verfahren (siehe z.B.: M. Loose et al: Real-time selective sequencing using nanopore technology, Nature Methods 2016, Vol. 13 No. 9, 751-758). Bei der Nanopore-Sequenzierung werden beispielsweise Nukleinsäuren von einem Enzym (Helicase) einzeln durch eine Pore in Nanometergröße transportiert, die sich in einer nicht leitfähigen Polymermembran befindet. Wird eine Spannung über diese Membran angelegt, so entsteht ein Ionenfluss durch die Pore, dessen Größe von den Basen in der Pore anhängig ist. Somit lassen sich durch die Messung des Ionenstroms während der Translokation der DNS/RNS durch die Pore sowohl die Basenabfolge als auch etwaige Modifikationen der Basen messen.

Die erfindungsgemäße Sequenzierung kann in mindestens zwei Phasen erfolgen, einer ersten Phase und einer zweiten Phase. Die erste Phase dient der Klärung, ob sich in der Probe überhaupt ein Pathogen befindet. Nur dann, wenn in der ersten Phase ein Pathogen identifiziert wird, findet die zweite Phase statt. Die zweite Phase dient der Identifizierung von Anzeichen einer Resistenz des identifizierten Pathogens gegenüber einem oder mehreren Bekämpfungsmitteln. In beiden Phasen werden Nukleinsäuren oder Peptide sequenziert und dabei Nukleotid-Abfolgen und Aminosäuren-Abfolgen ermittelt. In beiden Phasen werden die ermittelten Sequenzen während der Ermittlung mit Referenzsequenzen verglichen. In der ersten Phase erfolgt ein Vergleich der ermittelten Abfolgen mit mindestens einer Pathogen-Sequenz. Vorzugsweise erfolgt ein Vergleich mit einer Mehrzahl an Pathogen-Sequenzen. Kommt es zu einer Übereinstimmung zwischen einer ermittelten Nukleotid-Abfolge oder einer Aminosäuren-Abfolge und einer Pathogen-Sequenz, kann davon ausgegangen werden, dass das Pathogen in der Probe enthalten ist. Auf diese Weise kann ein Pathogen ermittelt werden, das in der Probe enthalten ist. In der zweiten Phase erfolgt ein Vergleich der ermittelten Abfolgen mit mindestens einem Resistenzmarker, der in der Vergangenheit bei dem ermittelten Pathogen beobachtet worden ist, und/oder mit einer resistenzfreien Sequenz. Dieser Vergleich dient der Identifizierung von Anzeichen für das Vorliegen einer Resistenz des ermittelten Pathogens gegenüber einem oder mehreren Bekämpfungsmitteln. Eine resistenzfreie Sequenz ist eine Nukleotid-Abfolge oder einer Aminosäuren-Abfolge eines Pathogens, die keine Anzeichen auf eine Resistenz des Pathogens gegenüber einem Bekämpfungsmittel aufweist. Bei der Pathogen-Sequenz, dem Resistenzmarker und der resistenzfreien Sequenz handelt es sich um Referenzsequenzen, die beispielsweise aus einer oder mehreren Datenbanken entnommen werden können.

Zunächst soll die erste Phase näher betrachtet werden.

Üblicherweise ist ein Großteil der in einer Probe vorhandenen Nukleinsäuren bzw. Peptide auf die Pflanze zurückzuführen, insbesondere, wenn es sich bei der Probe um einen Bestandteil einer Pflanze handelt. Die Bestimmung der Sequenz einer Pflanzen-Nukleinsäure oder eines Pflanzen-Proteins ist jedoch von sekundärem Interesse. Erfindungsgemäß wird daher die Sequenzierung einer Nukleinsäure oder eines Peptids in der ersten Phase der Sequenzierung abgebrochen, sobald sich herausstellt, dass die Nukleinsäure oder das Peptid nicht von einem Pathogen (sondern beispielsweise von der Pflanze) stammt.

Dazu werden während der Sequenzierung die Sequenzierungsergebnisse mit einer oder mehreren Pathogen-Sequenzen verglichen. Während also die Nukleotid-Abfolge in einer Nukleinsäure oder die Aminosäuren-Abfolge in einem Peptid festgestellt wird, wird jeweils geprüft, ob die Nukleotid-Abfolge bzw. die Aminosäure-Abfolge in einer oder mehreren Pathogen-Sequenzen enthalten ist. Dies sei an einem Beispiel erläutert. Es wird zunächst ein erstes Nukleotid in einer Nukleinsäure bestimmt, dann das zweite und so fort. Die Abfolge der Nukleotide wächst mit jedem weiteren ermittelten Nukleotid. Wenn ein weiteres Nukleotid der Abfolge bestimmt worden ist, wird geprüft, ob die (wachsende) Abfolge in einer oder mehreren Pathogen-Sequenzen enthalten ist. Eine Abfolge von beispielsweise vier Nukleotiden wird mit einer hohen Wahrscheinlichkeit an einer oder mehreren Stellen in einer Pathogen-Sequenz enthalten sein. Bei einer Abfolge von zwanzig Nukleotiden kann es jedoch bereits sein, dass diese Abfolge an keiner Stelle mehr in der Pathogen-Sequenz auftritt. Sobald sich herausstellt, dass eine Abfolge von Nukleotiden, die bei der Sequenzierung ermittelt wird, an keiner Stelle in einer oder mehreren Pathogen-Sequenzen enthalten ist, kann die Sequenzierung beendet werden; jede Bestimmung eines weiteren Nukleotids in der Nukleotid-Abfolge ist dann ohne Bedeutung; es ist bereits klar, dass die Nukleinsäure, die aktuell sequenziert wird, keine Nukleinsäure eines Pathogens sein kann. Dies gilt analog auch für die Bestimmung der Aminosäure-Abfolge in einem Peptid in der Probe.

Bei der mindestens einen Pathogen-Sequenz, die zum Abgleich in der ersten Phase der Sequenzierung verwendet wird, handelt es sich um eine Nukleotid-Sequenz bzw. Aminosäure-Sequenz eines definierten Pathogens, die für das Pathogen (oder eine definierte Gruppe von Pathogenen, z.B. Pathogene einer Art oder Gattung oder Unterfamilie oder Familie oder Unterordnung oder Ordnung oder Klasse) spezifisch sind. Anhand einer solchen spezifischen Pathogen-Sequenz kann damit auf ein spezifisches Pathogen (oder eine Gruppe von Pathogenen) geschlossen werden. Bei einer solchen Pathogen-Sequenz kann es sich beispielsweise um eine Nukleinsäure-Sequenz handeln, die die ITS-Region ganz oder teilweise umfasst. In jedem Fall muss es sich bei der Pathogen-Sequenz um eine Sequenz handeln, die sich von Sequenzen der Pflanze, die Gegenstand der Untersuchung ist, unterscheidet.

In einer bevorzugten Ausführungsform wird eine oder werden mehrere Pathogen-Sequenzen (automatisch) auf Basis von Informationen zur Pflanze, zum Zeitpunkt der Probennahme, zum Ort der Probennahme, zu den Umweltbedingungen (Temperatur, Luftdruck, Luftfeuchte, Niederschlagsmengen usw.), zum Zeitpunkt der Probennahme und/oder während einer definierten Zeitspanne vor dem Zeitpunkt der Probennahme (z.B. Wetter- und/oder Klimadaten) und/oder zur Historie (z.B. in der Vergangenheit beobachtete Pathogene und/oder Krankheiten) ausgewählt. Beispielsweise treten einige Krankheiten nur bei bestimmten Pflanzen auf. Wenn eine Probe von einer bestimmten Pflanze genommen wird, können Pathogen-Sequenzen von solchen Pathogenen ausgewählt werden, die diese Pflanze befallen und die Krankheiten auslösen können. Ferner treten Krankheitserreger üblicherweise nur in bestimmten Regionen und nur unter bestimmten Umweltbedingungen auf. Der Ort der Probennahme gibt Auskunft über die Region und die in der Region üblicherweise auftretenden Krankheitserreger. Die Umweltbedingungen zum Zeitpunkt der Probennahme und/oder in einer definierten Zeitspanne (z.B. vier Wochen oder drei Wochen oder zwei Wochen oder eine Woche oder ein Anzahl von Tagen) vor dem Zeitpunkt der Probennahme geben Auskunft darüber, welche Krankheitserreger überhaupt in Betracht zu ziehen sind bzw. welche Krankheitserreger ausgeschlossen werden können. Solche Krankheitserreger, die in der Vergangenheit bereits am Ort der Probennahme oder in der Nachbarschaft beobachtet worden sind (entweder direkt oder indirekt anhand von z.B. Krankheitssymptomen), können (wenn es die Umweltbedingungen und die jeweils betrachtete Pflanze erlauben) auch aktuell auftreten, weswegen eine spezifische Pathogen-Sequenz eines solchen Pathogens deswegen ausgewählt werden kann (und sollte), weil es (das Pathogen) in der Vergangenheit bereits aufgetreten ist.

Eine bevorzugte Ausführungsform der vorliegenden Erfindung umfasst damit die folgenden Schritte:
(1) Ermitteln einer Pflanze
(2) Ermitteln von mindestens einem Pathogen, das die Pflanze befallen kann (das in der Vergangenheit eine solche Pflanze befallen hat)
(3) Ermitteln von mindestens einer Pathogen-Sequenz für das mindestens eine ermittelte Pathogen
(4) Sammeln einer Probe von der Pflanze oder von einem Medium, in dem die Pflanze wächst
(5) Identifizieren eines Pathogens in der Probe
(6) Ermitteln von mindestens einem Resistenzmarker für das identifizierte Pathogen
(7) Identifizieren von Anzeichen auf eine Resistenz des identifizierten Pathogens gegenüber einem Bekämpfungsmittel.

Eine weitere bevorzugte Ausführungsform der vorliegenden Erfindung umfasst die folgenden Schritte:
(1) Ermitteln einer Pflanze
(2) Ermitteln von Umweltbedingungen, denen die Pflanze ausgesetzt ist und/oder ausgesetzt gewesen ist
(3) Ermitteln von mindestens einem Pathogen, das die Pflanze befallen kann (das in der Vergangenheit eine solche Pflanze befallen hat)
(4) Ermitteln von mindestens einer Pathogen-Sequenz für das mindestens eine ermittelte Pathogen
(5) Sammeln einer Probe von der Pflanze oder von einem Medium, in dem die Pflanze wächst
(6) Identifizieren eines Pathogens in der Probe
(7) Ermitteln von mindestens einem Resistenzmarker für das identifizierte Pathogen
(8) Identifizieren von Anzeichen auf eine Resistenz des identifizierten Pathogens gegenüber einem Bekämpfungsmittel.

Das Ermitteln der Pflanze und/oder das Ermitteln der Umweltbedingungen kann beispielsweise dadurch erfolgen, dass ein Nutzer die entsprechenden Informationen in das erfindungsgemäße System eingibt. Das Spezifizieren der Pflanze kann beispielsweise dadurch erfolgen, dass ein Nutzer den Namen der Pflanze oder den Namen der Sorte der Pflanze oder einen Code für die Pflanze/Pflanzensorte (z.B. nach dem Internationalen Code der Nomenklatur der Kulturpflanzen, kurz ICNCP) in das erfindungsgemäße System eingibt oder die entsprechende Information zu der Pflanze aus einer Liste oder einem Menü auswählt oder eine Pflanze anhand einer bildhaften Darstellung der Pflanze (z.B.: einem Foto oder eine Grafik) z.B. auf einem Monitor des erfindungsgemäßen Systems auswählt.

Denkbar ist auch, dass ein Nutzer eine Pflanze spezifiziert, die auf einem Feld angebaut wird und ggf. zusätzlich die Geokoordinaten des Feldes spezifiziert. Das erfindungsgemäße System kann konfiguriert sein, anhand der spezifizierten Pflanze und ggf. der Geokoordinaten in einer Datenbank Pathogene zu ermitteln, die bei der Pflanzenspezies auftreten können. Anhand der Geokoordinaten können beispielsweise aktuelle Wetterdaten und/oder Wetterdaten der Vergangenheit ermittelt werden. Es ist denkbar, dass für Pathogene, die bei der Pflanze auftreten können, Modelle ausgewählt werden, die anhand der Wetterdaten eine Wahrscheinlichkeit berechnen, dass diese Pathogene zum aktuellen Zeitpunkt bei der spezifizierten Pflanze auftreten. Es ist denkbar, dass nur solche Pathogene näher betrachtet werden, bei denen die Wahrscheinlichkeit, dass sie bei der spezifizierten Pflanze zum aktuellen Zeitpunkt auftreten, oberhalb eines Schwellenwerts (Mindestwahrscheinlichkeit) liegen. Der Schwellenwert kann empirisch ermittelt oder durch einen Nutzer willkürlich festgelegt werden.

Eine bevorzugte Ausführungsform der vorliegenden Erfindung umfasst damit die folgenden Schritte:
(1) Ermitteln einer Pflanze
(2) Ermitteln von mindestens einem Pathogen, das die Pflanze befallen kann (das in der Vergangenheit eine solche Pflanze befallen hat)
(3) Ermitteln von Umweltbedingungen, denen die Pflanze ausgesetzt ist oder ausgesetzt gewesen ist,
(4) Berechnen einer Wahrscheinlichkeit für das Auftreten des mindestens einen Pathogens bei der Pflanze zum aktuellen Zeitpunkt
(5) Vergleichen der Wahrscheinlichkeit mit einem vordefinierten Schwellenwert
(6) Ermitteln von Pathogen-Sequenzen für diejenigen ermittelten Pathogene, für die die Wahrscheinlichkeit oberhalb des Schwellenwerts liegt
(7) Sammeln einer Probe von der Pflanze oder von einem Medium, in dem die Pflanze wächst
(8) Identifizieren eines Pathogens in der Probe
(9) Ermitteln von mindestens einem Resistenzmarker für das identifizierte Pathogen,
(10) Identifizieren von Anzeichen auf eine Resistenz des identifizierten Pathogens gegenüber einem Bekämpfungsmittel.

Vorzugsweise liegen die eine oder die mehreren Pathogen-Sequenzen als FM-Index vor. Ein FM-Index ist ein komprimierter Volltext-Teilzeichenfolgenindex, der auf der Burrows-Wheeler-Transformation basiert und von P. Ferragina und G. Manzini entwickelt wurde (siehe z.B.: P. Ferragina und G. Manzini: Opportunistic Data Structures with Applications, Proceedings of the 41st Annual Symposium on Foundations of Computer Science 2000; 390-398). Der Name FM-Index steht für *Full-text index in Minute space.* Der FM-Index ist eine opportunistische Datenstruktur, die sowohl eine Komprimierung des Eingabetextes als auch eine schnelle Teilzeichenfolgenabfrage ermöglicht. Ein FM-Index kann verwendet werden, um die Anzahl der Vorkommen eines Musters innerhalb des komprimierten Eingabetextes effizient zu ermitteln und die Position jedes Vorkommens zu lokalisieren. Die Abfragezeit sowie der erforderliche Speicherplatz sind in Bezug auf die Größe der Eingabedaten sublinear komplex.

Der Vergleich der gerade ermittelten Nukleotid-Abfolge bzw. Aminosäuren-Abfolge mit einer oder mehreren Pathogen-Sequenzen kann von einer Steuer- und Berechnungseinheit vorgenommen werden. Bei einer solchen Steuer- und Berechnungseinheit kann es sich um einen oder mehrere Computer handeln. Die Steuer- und Berechnungseinheit kann ein Bestandteil der Sequenziereinheit oder eine separate Einheit sein. Die Steuer- und Berechnungseinheit hat Zugriff auf einen oder mehrere Datenspeicher. In dem mindestens einen Datenspeicher ist mindestens eine Pathogen-Sequenz gespeichert. Die Steuer- und Berechnungseinheit lädt die mindestens eine Pathogen-Sequenz in einen Arbeitsspeicher, empfängt die Nukleotid-Abfolge während ihrer Ermittlung (Nukleotid für Nukleotid oder in Blöcken von mehreren Nukleotiden) und prüft, ob die (wachsende) Nukleotid-Abfolge ein- oder mehrfach in der Pathogen-Sequenz vorhanden ist.

Die Technologie, eine (wachsende) Abfolge von Nukleotiden in einer Sequenz während ihrer Bestimmung für einen Vergleich zu verwenden, ist im Stand der Technik beschrieben (siehe z.B.: S. Kovaka et al.: Targeted nanopore sequencing by real-time mapping of raw electrical signal with UNCALLED, https://doi.org/10.1101/2020.02.03.931923).

Sobald sich herausstellt, dass die ermittelte Abfolge von Nukleotiden bzw. Aminosäuren in der mindestens einen Pathogen-Sequenz an keiner Stelle auftaucht, wird die Sequenzierung des jeweiligen Moleküls abgebrochen und damit die weitere Sequenzierung des Moleküls unterbunden. Die Technologie, eine Bestimmung einer Nukleotid-Abfolge abzubrechen, ist im Stand der Technik beschrieben (siehe z.B.: A. Payne et al.: Nanopore adaptive sequencing for mixed samples, whole exome capture and targeted panels, https://doi.org/10.1101/2020.02.03.926956). Im Fall der Nanopore-Technologie kann beispielsweise mit Hilfe eines Spannungspulses eine Nukleinsäure, die durch eine Pore durchgeschleust wird, aus der Pore befördert werden, und so die weitere Sequenzierung abgebrochen werden.

Wird innerhalb einer vordefinierten Zeitspanne keine Nukleinsäure oder kein Peptid in der Probe sequenziert, die/das eine Pathogen-Sequenz aufweist, kann davon ausgegangen werden, dass sich in der Probe kein Pathogen befindet. Die vordefinierte Zeitspanne kann empirisch ermittelt werden. Es kann eine Mitteilung ausgegeben werden, dass in der Probe kein Pathogen gefunden wurde.

Sobald sich herausstellt, dass die Sequenz einer Nukleinsäure oder eines Peptids in der Probe mit einer spezifischen Pathogen-Sequenz übereinstimmt, kann angenommen werden, dass sich das entsprechende Pathogen in der Probe befindet. Aus der Kenntnis der Pathogen-Sequenz ergibt sich also automatisch das jeweilige Pathogen. Für das so ermittelte Pathogen wird anhand einer Datenbank geprüft, ob es bekannte Resistenzen gegenüber einem oder mehreren Bekämpfungsmitteln gibt. "Bekannt" bedeutet, dass eine entsprechende Resistenz in der Vergangenheit bei einer Population des Pathogens beobachtet worden ist. Falls mindestens eine Resistenz beobachtet worden ist, wird mindestens ein Resistenzmarker ermittelt.

Unter einem "Resistenzmarker" wird eine genetische Information verstanden, die Auskunft darüber gibt, ob ein Schadorganismus eine Resistenz gegenüber einem Bekämpfungsmittel entwickeln könnte, gerade entwickelt oder entwickelt hat.

Es ist für eine Vielzahl von Krankheitserregern bekannt, welche Sequenzen anzeigen, dass die Krankheitserreger eine Resistenz gegenüber einem Bekämpfungsmittel entwickeln oder aufweisen (siehe zum Beispiel: S. Omrane et al.: Plasticity of the MFS1 Promoter Leads to Multidrug Resistance in the Wheat Pathogen Zymoseptoria tritici, https://doi.org/10.1128/mSphere.00393-17; Y. Choi et al.: Identification of Genes Related to Fungicide Resistance in Fusarium fujikuroi, Mycobiology. 2017, 45(2): 101-104; K. J. Brent: Fungicie Resistance in crop pathogens: How can it be managed? ISBN 90-72398-07-6; K. G. Zulak et al.: Improved Detection and Monitoring of Fungicide Resistance in Blumeria graminis f. sp. hordei With High-Throughput Genotype Quantification by Digital PCR, Front Microbiol. 2018, 9: 706; M. de miccolis et al: Genetics of Fungicide Resistance, DOI: 10.1007/978-4-431-55642-8_2; H. B. Deising et al.: Mechanisms and significance of fungicide resistance, Braz. J. Microbiol. 2008, Vol.39 No.2; L. Kanetis at al.: Fungicide resistance profile and genetic structure of Botrytis cinerea from greenhouse crops in Cyprus, Eur. J. Plant. Pathol. 2017, 147, 527-540; J. L. Beckerman: Detection of Fungicide Resistance, DOI: 10.5772/55981; L. Yang et al.: Cross-resistance of the pathogenic fungus Alternaria alternata to fungicides with different modes of action, BMC Microbiol 2019, 19, 205).

Ein "Resistenzmarker" ist also eine Sequenz von Nukleotiden oder eine Sequenz von Aminosäuren, die in Nukleinsäuren bzw. Peptiden von resistenten Populationen gefunden wurde, und die für die Resistenz verantwortlich ist bzw. die Resistenz begünstigt. Es kann sich dabei beispielsweise um die Target-Sequenz in einem resistenten Pathogen handeln, die gegenüber nicht-resistenten Spezies derselben Art eine Mutation aufweist. Dabei definiert das "Target" den Wirkort des Bekämpfungsmittels beim Pathogen. Eine genetische Veränderung der Target-Sequenz kann bewirken, dass das Bekämpfungsmittel nicht mehr oder nur noch vermindert wirkt.

Ist keine Resistenz für das identifizierte Pathogen bekannt, kann eine Mitteilung ausgegeben werden, dass in der Probe ein Pathogen identifiziert worden ist, um welches Pathogen es sich handelt, und, dass für das Pathogen keine Resistenz bekannt ist.

Sobald ein Pathogen in der Probe identifiziert ist, und mindestens ein Resistenzmarker für das Pathogen ermittelt ist, wird die zweite Phase der Sequenzierung eingeleitet.

In der zweiten Phase wird die Sequenzierung der in der Probe befindlichen Nukleinsäuren oder Peptide fortgesetzt; in der zweiten Phase liegt der Fokus jedoch auf der Identifizierung von Anzeichen einer Resistenz des identifizierten Pathogens gegenüber einem oder mehreren Bekämpfungsmitteln. Dazu werden die bei der Sequenzierung ermittelten Abfolgen der Nukleotide bzw. Aminosäuren mit dem mindestens einen Resistenzmarker und/oder mit einer resistenzfreien Sequenz verglichen.

Treten bei der Sequenzierung keine Abfolgen von Nukleotiden oder Aminosäuren auf, die mit der Sequenz des mindestens einen Resistenzmarkers übereinstimmen, so kann davon ausgegangen werden, dass das in der Probe identifizierte Pathogen keine bekannte Resistenz aufweist. Es kann eine Mitteilung ausgegeben werden, dass ein Pathogen gefunden wurde, um welches Pathogen es sich handelt, und dass keine Anzeichen für das Vorliegen einer bekannten Resistenz gegenüber einem Bekämpfungsmittel gefunden wurden. Treten aber Abweichungen gegenüber der resistenzfreien Sequenz auf, kann es sein, dass eine (noch) unbekannte Resistenz vorliegt und/oder dass das Pathogen eine (noch unbekannte) Resistenz entwickelt. In dem Fall kann eine Mitteilung ausgegeben werden, dass ein Pathogen gefunden wurde, um welches Pathogen es sich handelt, und dass keine Anzeichen für das Vorliegen einer bekannten Resistenz gegenüber einem Bekämpfungsmittel gefunden wurden, aber dass Abweichungen in der Abfolge von Nukleotiden oder Aminosäuren vorliegen gegenüber der resistenzfreien Sequenz vorliegen, die näher betrachtet werden können/sollten.

Treten bei der Sequenzierung Abfolgen von Nukleotiden oder Aminosäuren auf, die mit der Sequenz eines Resistenzmarkers übereinstimmen, so ist dies ein Anzeichen für das Vorliegen einer (bekannten) Resistenz. Es kann eine Mitteilung ausgegeben werden, die angibt, dass ein Pathogen in der Probe gefunden wurde, dass das Pathogen Anzeichen einer Resistenz aufweist und es kann die Resistenz benannt werden, z.B. anhand des übereinstimmenden Resistenzmarkers.

In einer bevorzugten Ausführungsform umfasst der Schritt "Identifizieren von Anzeichen auf eine Resistenz des identifizierten Pathogens gegenüber einem Bekämpfungsmittel" damit die folgenden Teilschritte:
(1) Vergleichen der Sequenzen von den Nukleinsäuren oder Peptiden in der Probe mit dem mindestens einen ermittelten Resistenzmarker und/oder einer resistenzfreien Sequenz,
(2) für den Fall, dass der Resistenzmarker in keiner der Sequenzen von den Nukleinsäuren oder Peptiden in der Probe auftritt und Sequenzen der Probe der resistenzfreien Sequenz entsprechen: Ausgeben einer Mitteilung, dass für das identifizierte Pathogen kein Anzeichen einer Resistenz gefunden wurde,
(3) für den Fall, dass der Resistenzmarker in keiner der Sequenzen von den Nukleinsäuren oder Peptiden in der Probe auftritt und die Sequenzen der Probe nicht der resistenzfreien Sequenz entsprechen: Ausgeben einer Mitteilung, dass für das identifizierte Pathogen kein Anzeichen einer bekannten Resistenz gefunden wurde, dass aber Abweichungen gegenüber der resistenzfreien Sequenz gefunden wurden,
(4) für den Fall, dass der Resistenzmarker in einer oder in mehreren der Sequenzen auftritt: Ausgeben einer Mitteilung, dass für das identifizierte Pathogen ein Anzeichen einer Resistenz gefunden wurde, und Ausgeben einer Mitteilung über die Resistenz.

Es ist denkbar, dass sich die Sequenz einer Nukleinsäure bzw. eines Peptids von einem resistenten Pathogen nur um ein Nukleotid oder wenige Nukleotide bzw. nur um eine Aminosäure oder wenige Aminosäuren von der entsprechenden Sequenz eines nicht-resistenten Pathogens unterscheidet. Auf der anderen Seite kann es bei der Sequenzierung von einzelnen Nukleinsäuren oder Peptiden immer zu Fehlern kommen. Wird daher eine Nukleinsäure oder ein Peptid identifiziert, die/das eine Sequenz gemäß einem Resistenzmarker aufweist, ist es grundsätzlich möglich, dass es sich hierbei um einen Fehler handelt. Daher ist es denkbar, dass erst dann von dem Vorliegen eines Anzeichens einer Resistenz ausgegangen wird, wenn eine Mindestzahl an Nukleinsäuren oder Aminosäuren gefunden wurde, die eine Sequenz gemäß einem Resistenzmarker aufweist.

In einer bevorzugten Ausführungsform umfasst der Schritt "Identifizieren von Anzeichen auf eine Resistenz des identifizierten Pathogens gegenüber einem Bekämpfungsmittel" damit die folgenden Teilschritte:
(1) Vergleichen der Sequenzen von den Nukleinsäuren oder Peptiden in der Probe mit dem mindestens einen ermittelten Resistenzmarker,
(2) für den Fall, dass der Resistenzmarker in keiner der Sequenzen von den Nukleinsäuren oder Peptiden in der Probe auftritt oder in einer Anzahl von Nukleinsäuren auftritt, die kleiner als eine vordefinierte Mindestanzahl ist: Ausgeben einer Mitteilung, dass für das identifizierte Pathogen kein Anzeichen einer Resistenz gefunden wurde,
(3) für den Fall, dass der Resistenzmarker in einer Mindestanzahl der Sequenzen auftritt oder in einer Anzahl, die größer als die Mindestanzahl ist: Ausgeben einer Mitteilung, dass für das identifizierte Pathogen ein Anzeichen einer Resistenz gefunden wurde, und Ausgeben einer Mitteilung über die Resistenz.

In einer bevorzugten Ausführungsform der vorliegenden Erfindung werden die Sequenzen von Nukleinsäuren oder Peptiden in einer Probe in der zweiten Phase der Sequenzierung nicht (nur) mit Resistenzmarkern verglichen, sondern auch mit der entsprechenden Sequenz eines nicht-resistenten Pathogens derselben Art. Diese Sequenz wird in dieser Beschreibung auch als resistenzfreie Sequenz bezeichnet. Auch die resistenzfreie Sequenz ist eine Referenzsequenz.

Es ist denkbar, dass Nukleinsäuren oder Peptide in der Probe gefunden werden, die von einem Pathogen stammen, wobei deren Sequenz aber weder dem Resistenzmarker noch der resistenzfreien Sequenz entspricht. Es kann sein, dass es sich um eine neue Mutation handelt, die ggf. zu einer noch unbekannten Resistenz führen könnte. Durch einen Abgleich der Sequenzen von den Nukleinsäuren oder Peptiden in der Probe mit der resistenzfreien Sequenz können also neue, sich ggf. entwickelnde Resistenzen frühzeitig identifiziert werden.

Eine bevorzugte Ausführungsform der vorliegenden Erfindung umfasst damit die folgenden Schritte:
- Sammeln einer Probe von einer Pflanze oder von einem Medium, in dem die Pflanze wächst
- Identifizieren eines Pathogens in der Probe
- Ermitteln von mindestens einem Resistenzmarker für das identifizierte Pathogen und Ermitteln einer resistenzfreien Sequenz für das identifizierte Pathogen
- Identifizieren von Anzeichen auf eine Resistenz des identifizierten Pathogens gegenüber einem Bekämpfungsmittel durch Vergleichen der Sequenzen von Nukleinsäuren oder Peptiden in der Probe mit dem mindestens einen ermittelten Resistenzmarker und der resistenzfreien Sequenz
- für den Fall, dass eine Sequenz oder eine Anzahl an Sequenzen, die größer oder gleich einer Mindestanzahl ist, weder eine Übereinstimmung mit dem mindestens einen Resistenzmarker noch mit der resistenzfreien Sequenz aufweist: Ausgeben einer Mitteilung, dass in der Probe ein Pathogen identifiziert wurde, das eine Mutation aufweist, die auf eine neue und/oder sich entwickelnde Resistenz hindeutet.

Auch für den Fall, dass in einer Probe ein Pathogen nachgewiesen werden konnte, aber für das Pathogen keine Resistenz bekannt ist, kann die zweite Phase der Sequenzierung eingeleitet werden, z.B. um die Target-Sequenz beim Pathogen zu analysieren und ggf. Mutationen zu identifizieren, die auf eine sich entwickelnde Resistenz hinweisen. In einem solchen Fall werden die ermittelten Abfolgen von Nukleotiden oder Aminosäuren nicht mit dem Resistenzmarker, sondern mit der Target-Sequenz vergleichen. Auch die Target-Sequenz ist eine Referenzsequenz. Die Target-Sequenz, die resistenzfreie Sequenz und der Referenzmarker werden in dieser Beschreibung auch als resistenzanzeigende Sequenz bezeichnet, da anhand dieses Sequenzabschnitts eine Resistenz oder die Abwesenheit einer Resistenz festgestellt werden kann.

Eine bevorzugte Ausführungsform der vorliegenden Erfindung umfasst damit die folgenden Schritte:
- Sammeln einer Probe von einer Pflanze oder von einem Medium, in dem die Pflanze wächst
- Identifizieren eines Pathogens in der Probe
- Ermitteln einer Target-Sequenz für das identifizierte Pathogen
- Identifizieren von Anzeichen auf eine neue Resistenz des identifizierten Pathogens gegenüber einem Bekämpfungsmittel durch Vergleichen der Sequenzen von Nukleinsäuren oder Peptiden in der Probe mit der Target-Sequenz
- für den Fall, dass eine Sequenz oder eine Anzahl an Sequenzen, die größer oder gleich einer Mindestanzahl ist, eine Mutation der Target-Sequenz darstellt: Ausgeben einer Mitteilung, dass in der Probe ein Pathogen identifiziert wurde, das eine Mutation aufweist, die auf eine neue und/oder sich entwickelnde Resistenz hindeutet.

In einer bevorzugten Ausführungsform wird die Häufigkeit *Q_{R}* von in einer Probe gefundenen Nukleinsäuren oder Peptiden mit einer Sequenz gemäß dem Resistenzmarker ermittelt, und es wird die Häufigkeit *Q_{NR}* an in der Probe gefundenen Nukleinsäuren oder Peptiden mit einer Sequenz gemäß der resistenzfreien Sequenz ermittelt. Die ermittelten Häufigkeiten *Q_{R}* und *Q_{NR}* können zueinander ins Verhältnis gesetzt werden (z.B. *Q_{R}* / *Q_{NR}*)*.* Es ist denkbar, dass erst dann von dem Vorliegen eines Anzeichens einer Resistenz ausgegangen wird, wenn das Verhältnis einen vordefinierten Mindestwert überschritten hat. Der Mindestwert kann empirisch ermittelt werden.

Eine bevorzugte Ausführungsform der vorliegenden Erfindung umfasst damit die folgenden Schritte:
- Sammeln einer Probe von einer Pflanze oder von einem Medium, in dem die Pflanze wächst
- Identifizieren eines Pathogens in der Probe
- Ermitteln von mindestens einem Resistenzmarker für das identifizierte Pathogen und Ermitteln einer resistenzfreien Sequenz für das identifizierte Pathogen
- Vergleichen der Sequenzen von Nukleinsäuren oder Peptiden in der Probe mit dem mindestens einen ermittelten Resistenzmarker und der resistenzfreien Sequenz
- Ermitteln einer Häufigkeit *Q_{R}* von in der Probe gefundenen Nukleinsäuren oder Peptiden mit einer Sequenz gemäß dem Resistenzmarker, Ermitteln einer Häufigkeit *Q_{NR}* an in der Probe gefundenen Nukleinsäuren oder Peptiden mit einer Sequenz gemäß der resistenzfreien Sequenz, Berechnen eines Verhältnisses der ermittelten Häufigkeiten *Q_{R}* und *Q_{NR},* und Vergleichen des Verhältnisses mit einem vordefinierten Mindestwert
- für den Fall, dass das Verhältnis größer oder gleich den Mindestwert ist: Ausgeben einer Mitteilung, dass für das identifizierte Pathogen ein Anzeichen einer Resistenz gefunden wurde, und Ausgeben einer Mitteilung über die Resistenz.

Für den Vergleich der Nukleinsäure-Sequenzen oder Peptid-Sequenzen mit dem mindestens einen Resistenzmarker und/oder der resistenzfreien Sequenz und/oder der Target-Sequenz kann ansonsten analog das gelten, was in dieser Beschreibung für den Vergleich der Nukleinsäure-Sequenzen oder Peptid-Sequenzen mit der mindestens einen Pathogen-Sequenzen gilt:
- Um auch die zweite Phase der Sequenzierung möglichst effizient durchzuführen, und um zu verhindern, dass ein Großteil der Sequenzierung auf Nukleinsäuren oder Peptide der Pflanze oder Nicht-Targetsequenzen des Pathogens fallen, kann auch in der zweiten Phase der Sequenzierung die Sequenzierung einzelner Nukleinsäuren oder Peptide abgebrochen werden, sobald sich herausstellt, dass eine gerade sequenzierte Nukleinsäure oder ein gerade sequenziertes Peptid nicht von einer Targetsequenz desPathogens stammen kann (weil die ermittelte Abfolge in keiner Pathogen-Targetsequenz auftritt).
- Der mindestens eine Resistenzmarker und/oder die resistenzfreie Sequenz liegen vorzugsweise als FM-Index vor.
- Die Steuer- und Berechnungseinheit kann den mindestens einen Resistenzmarker und/oder die resistenzfreie Sequenz in einen Arbeitsspeicher laden, und die (wachsende) Nukleotid-Abfolge bzw. Aminosäuren-Abfolge während ihrer Ermittlung empfangen und mit dem mindestens einen Resistenzmarker und/oder mit der resistenzfreien Sequenz vergleichen.

Ausgaben von Informationen und/oder Mitteilungen bei der Ausführung der vorliegenden Erfindung können beispielsweise in Form von Bildern und/oder Text und/oder Sprache erfolgen. Informationen und/oder Mitteilungen können auf einem Bildschirm (Monitor) angezeigt, über einen Drucker ausgedruckt, in einem Datenspeicher gespeichert und/oder über ein Netzwerk an einen anderen Computer übermittelt werden.

Eine Information über ein Pathogen, das identifiziert worden ist, kann beispielsweise der Name des Pathogens sein. Es können auch Informationen ausgegeben werden, in welcher Probe das Pathogen gefunden worden ist, und/oder an welchem Ort die Probe gesammelt worden ist und/oder zu welchem Zeitpunkt die Probennahme erfolgt ist und/oder von welcher Pflanze die Probe genommen worden ist.

Eine Information über eine Resistenz, die identifiziert worden ist, kann den Namen der Resistenz, das von der Resistenz betroffene Target und/oder den Namen eines oder mehrerer Bekämpfungsmittel oder eines oder mehrerer Wirkstoffe, gegen das/die das identifizierte Pathogen resistent ist, umfassen.

Das Ergebnis der erfindungsgemäßen Analyse der Probe einer Pflanze kann vielfältig genutzt werden. Für den Fall, dass ein Pathogen identifiziert worden ist, bei dem keine Anzeichen für das Vorliegen einer Resistenz oder das Aufkommen einer neuen Resistenz gefunden wurden, kann ein Bekämpfungsmittel oder können mehrere Bekämpfungsmittel genannt werden, die zur Bekämpfung des Pathogens eingesetzt werden können. Für den Fall, dass ein Pathogen identifiziert worden ist, bei dem Anzeichen für das Vorliegen einer bekannten Resistenz gefunden wurden, können Bekämpfungsmittel genannt werden, bei denen die Resistenz nicht zum Tragen kommt. Ein solches Bekämpfungsmittel kann dann auch der Einsatz von elektrischer Energie, thermischer Energie (z.B. in Form von Wasserdampf oder Abflammen), elektromagnetischer Strahlung (UV-Strahlung, Laserbestrahlung) und/oder mechanischen Mitteln zum Entfernen der befallenen Pflanze oder Pflanzenteilen sein.

Da die Erfindung auch von einem autonomen System vor Ort im Feld ausgeführt werden kann, kann das erfindungsgemäße System auch eine Bekämpfungsvorrichtung zum Bekämpfen von Krankheitserregern umfassen, die nach (automatisierter) Auswahl des Bekämpfungsmittels zur Bekämpfung des identifizierten Pathogens automatisiert das ausgewählte Bekämpfungsmittel anwenden kann.

Das Ergebnis der erfindungsgemäßen Analyse auf Pathogene und Resistenzen kann ferner in einer Pathogenkarte festgehalten werden. Eine solche "Pathogenkarte" ist eine Repräsentation eines Teils der Erdoberfläche, in der für eine Mehrzahl an Orten auf der Erdoberfläche Informationen darüber festgehalten werden, ob an dem entsprechenden Ort ein Pathogen beobachten worden ist, und ob bei dem Pathogen ein Anzeichen auf eine (bestehende oder sich entwickelnde oder mögliche) Resistenz gegenüber einem Bekämpfungsmittel gefunden wurde. Vorzugsweise handelt es sich bei der Pathogenkarte um eine digitale Pathogenkarte.

Der Begriff "digital" bedeutet, dass die Karte von einer Maschine, in der Regel einem Computersystem, verarbeitet werden kann.

Unter "Verarbeitung" werden die bekannten Verfahren zur elektronischen Datenverarbeitung (EDV) verstanden.

Vorzugsweise ist die digitale Pathogenkarte eine digitale Repräsentation eines Feldes oder eines Feldes inklusive benachbarter Felder oder einer Region. Vorzugsweise werden separate Pathogenkarten für einzelne Pathogene und/oder für einzelne Bekämpfungsmittel oder Gruppen von Bekämpfungsmitteln, die den gleichen Wirkstoff oder die gleiche chemische/biologische Klasse (z.B. eine chemische Strukturklasse) von Wirkstoffen oder denselben Wirkmechanismus oder denselben Wirkort (Target) aufweisen, und/oder für spezifische Pflanzen, bei denen das Pathogen auftreten kann, erzeugt. Vorzugsweise lassen sich separate digitale Pathogenkarten miteinander verknüpfen, d.h. virtuell übereinanderlegen.

In einer bevorzugten Ausführungsform ist/sind für jeden Ort auf der digitalen Pathogenkarte, für den ein oder mehrere Analysenergebnisse vorliegen, der Zeitpunkt oder die Zeitpunkte hinterlegt, an dem/denen die jeweilige Analyse durchgeführt worden ist.

In einer bevorzugten Ausführungsform sind mehrere digitale Pathogenkarten so miteinander verknüpft, dass sie eine zeitliche Entwicklung der Verteilung einer oder mehrerer Resistenzen aufzeigen.

Vorzugsweise lassen sich digitale Pathogenkarten mit anderen digitalen Karten kombinieren; zum Beispiel mit digitalen Karten zur Bodenbeschaffenheit, zum Wasserhaushalt, zu angebauten Kulturpflanzen, zu Temperaturen (zu definierten Zeitpunkten und/oder für definierten Zeitspannen zum Beispiel in Form von mittleren und/oder minimalen und/oder maximalen Temperaturen), zu Niederschlagsmengen (zu definierten Zeitpunkten und/oder für definierten Zeitspannen zum Beispiel in Form von mittleren und/oder minimalen und/oder maximalen Niederschlagsmengen), zur Sonneneinstrahlung, zu Luftmassenbewegungen (Windrichtungen und Windstärken), zu in der Vergangenheit aufgetretenen Befällen mit einem oder mehreren Krankheitserregern, zu landwirtschaftlichen Maßnahmen, die vorgenommen worden sind (z.B. Aussaat, Bewässerung, Pflügen, Applikation von Pflanzenschutzmitteln, Nährstoffgabe, und dergleichen) usw. Die Werte von den Parametern, die in einer digitalen Karte festgehalten werden, können gemessene und/oder vorhergesagte Werte sein.

Eine Pathogenkarte kann zum Beispiel einem Landwirt wertvolle Informationen darüber geben, in welchen Bereichen seines Feldes (oder seiner Felder) Pathogene vorhanden sind und welche Resistenzen vorhanden sind oder sich ausbreiten. Es können Verbreitungsmuster analysiert werden, um die Ursachen für die Entstehung und/oder Verbreitung von Pathogenen und/oder Resistenzen zu erkunden. Bei mehreren zeitlich aufeinander folgenden Analysen gewinnt der Landwirt Einblick in die Ausbreitung von Pathogenen und/oder Resistenzen. Er kann dann Maßnahmen ergreifen, um die Pathogene und/oder Resistenzen zu bekämpfen. In einer Pathogenkarte kann ein Landwirt auch erkennen, ob in Nachbarfeldern ein Befall mit (resistenten) Pathogenen beobachtet worden ist. Die Informationen können ihm helfen, präventive Maßnahmen für seine Felder zu ergreifen.

Darüber hinaus erlauben detailliertere, nahezu in Echtzeit generierte digitale Pathogenkarten in Kombination mit weiteren Daten, wie z.B. Sonneneinstrahlung, Temperatur, Luftfeuchte und Windrichtung, eine genauere Vorhersage zur Ausbreitung von resistenten und nicht-resistenten Pathogenen. Dies erlaubt eine gezielte Bekämpfung, bzw. prophylaktische Behandlung angrenzender Gebiete, sowie einen gezielteren Einsatz von Bekämpfungsmitteln.

Weiterhin erlaubt die gezielte Erfassung genetischer Informationen von Pathogenpopulationen in der Umwelt eine Abschätzung einer bevorstehenden Resistenzentwicklung gegen ein bestimmtes Bekämpfungsmittel.

In einem weiteren Schritt des erfindungsgemäßen Verfahrens kann eine Maßnahme zur Bekämpfung eines Krankheitserregers und/oder einer (sich entwickelnden) Resistenz (auf Basis der Pathogenkarte) empfohlen und/oder durchgeführt werden. Zunächst einmal können anhand des Pathogens, das in der Probe identifiziert worden ist, eine oder mehrere Bekämpfungsmittel zur Bekämpfung des Pathogens identifiziert werden. Dies kann beispielsweise durch eine Abfrage in einer oder mehreren Datenbanken erfolgen. In der einen oder den mehreren Datenbanken können Informationen über Bekämpfungsmittel und die Pathogene, gegen die sie eingesetzt werden, gespeichert sein. Für den Fall, dass das identifizierte Pathogen eine Resistenz aufweist, kann in einem zweiten Schritt geprüft werden, ob die Resistenz gegenüber einem oder mehreren der aus der mindestens einen Datenbank ermittelten Bekämpfungsmitteln besteht. Es kann dasjenige Bekämpfungsmittel oder es können diejenigen Bekämpfungsmittel identifiziert werden, gegenüber das/die das identifizierte Pathogen nicht resistent ist. Es kann dann eine Behandlung der Pflanzen mit dem (einem der) identifizierten Bekämpfungsmittel(n) erfolgen. Sollte das Pathogen gegenüber allen aus der mindestens einen Datenbank ermittelten Bekämpfungsmitteln resistent sein, dann können alternative Maßnahmen zur Bekämpfung der Pathogene und/oder Ausbreitung einer Krankheit identifiziert, vorgeschlagen und/oder ausgeführt werden, wie beispielsweise: vollständiges mechanisches Entfernen der betroffenen Pflanzen, Fruchtwechsel, Abflammen, Zerstören durch Kälte, Zerstören durch elektrische Energie, Zerstören durch elektromagnetische Energie (z.B. mittels UV-Licht, Laserlicht) und/oder dergleichen.

Die vorliegende Erfindung kann ganz oder teilweise mit einem Computersystem ausgeführt werden. Ein "Computersystem" ist ein System zur elektronischen Datenverarbeitung, das mittels programmierbarer Rechenvorschriften Daten verarbeitet. Ein solches System umfasst üblicherweise eine Steuer- und Berechnungseinheit, oftmals auch als "Computer" bezeichnet, diejenige Einheit, die einen Prozessor zur Durchführung logischer Operationen und einen Arbeitsspeicher zum Laden eines Computerprogramms umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Joystick, Laufwerke, Kamera, Mikrofon, Lautsprecher etc. Auch interne Anschlüsse und Erweiterungskarten gelten in der Computertechnik als Peripherie.

Computersysteme von heute werden häufig in Desktop-PCs, Portable PCs, Laptops, Notebooks, Netbooks, Tablet-PCs und so genannte Handhelds (z.B. Smartphone) unterteilt; alle diese Systeme können zur Ausführung der Erfindung genutzt werden.

Eingaben in das Computersystem (zum Bespiel zur Steuerung durch einen Nutzer) erfolgen über Eingabemittel wie beispielsweise eine Tastatur, eine Maus, ein Mikrofon, ein berührungsempfindliches Display und/oder dergleichen. Ausgaben erfolgen über eine oder mehrere Ausgabeeinheiten, die insbesondere ein Monitor (Bildschirm), ein Drucker und/oder ein Datenspeicher sein kann/können.

Die Erfindung wird nachfolgend anhand von konkreten Ausführungsbeispielen und Figuren näher erläutert, ohne die Erfindung auf diese Beispiele, die in den Beispielen gezeigten Merkmale und/oder Kombinationen von Merkmalen beschränken zu wollen.

Es zeigen:

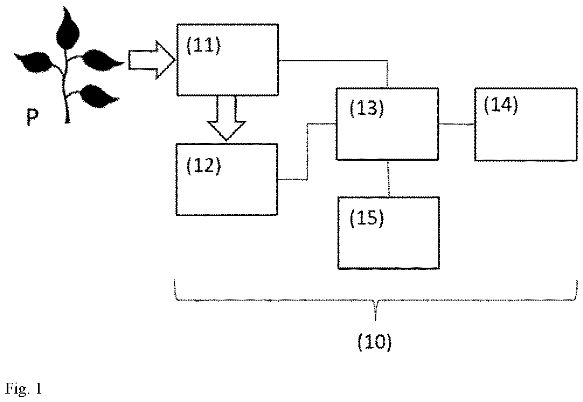

Figur 1 zeigt schematisch eine Ausführungsform des erfindungsgemäßen Systems. Das System (10) umfasst eine Probennahmeeinheit (11), eine Sequenziereinheit (12), eine Steuer- und Berechnungseinheit (13), einen Datenspeicher (14) und eine Ausgabeeinheit (15).

Mittels der Probennahmeeinheit (11) kann eine Probe von einer Pflanze P oder von einem Medium, in dem die Pflanze P wächst, genommen werden. Die Probe kann aufbereitet werden und der Sequenziereinheit (12) zugeführt werden.

Mittels der Sequenziereinheit (12) werden Sequenzen von Nukleinsäuren, die sich in der Probe befinden, ermittelt.

Die Steuer- und Berechnungseinheit (13) dient der Steuerung der einzelnen Komponenten des erfindungsgemäßen Systems (10) und der Koordinierung der Daten- und Signalflüsse. Bei der Steuer- und Berechnungseinheit kann es sich um einen Universalcomputer (engl. *general purpose computer)* handeln, der mittels des erfindungsgemäßen Computerprogramms entsprechend konfiguriert worden ist, die hier beschriebenen Schritte auszuführen.

Die Steuer- und Berechnungseinheit (13) ist mit dem Datenspeicher (14) verbunden, von dem sie eine oder mehrere Pathogen-Sequenzen und eine oder mehrere resistenzanzeigende Sequenzen für Vergleichszwecke abrufen kann.

Die Steuer- und Berechnungseinheit (13) empfängt wachsende Sequenzen von Nukleinsäuren von der Sequenziereinheit (12).

In einer ersten Phase prüft die Steuer- und Berechnungseinheit (13), ob die wachsenden Sequenzen in einer oder mehreren Pathogen-Sequenzen enthalten sind. Sobald sich herausstellt, dass eine wachsende Sequenz einer Nukleinsäure nicht in der mindestens einen Pathogen-Sequenz enthalten ist, sendet die Steuer- und Berechnungseinheit (13) ein Signal an die Sequenziereinheit (12). Durch das Signal wird die Sequenzierung der Nukleinsäure abgebrochen und es wird die Sequenzierung einer neuen Nukleinsäure gestartet. Sobald sich herausstellt, dass die Sequenz einer Nukleinsäure mit einer Pathogen-Sequenz übereinstimmt, veranlasst die Steuer- und Berechnungseinheit (13) die Ausgabeeinheit (15) zum Ausgeben von Informationen über das Pathogen, mit dessen Sequenz die Sequenz der Nukleinsäure übereinstimmt. Ferner wechselt die Steuer- und Berechnungseinheit (13) in eine zweite Phase.

In der zweiten Phase prüft die Steuer- und Berechnungseinheit (13), ob die wachsenden Sequenzen der Nukleinsäuren in der Probe eine Übereinstimmung mit einem Resistenzmarker, einer resistenzfreien Sequenz und/oder einer Target-Sequenz aufweisen. Sobald sich herausstellt, dass die Sequenz einer Nukleinsäure mit einer resistenzanzeigenden Sequenz übereinstimmt, veranlasst die Steuer- und Berechnungseinheit (13) die Ausgabeeinheit (15) zum Ausgeben von Informationen über die Resistenz.

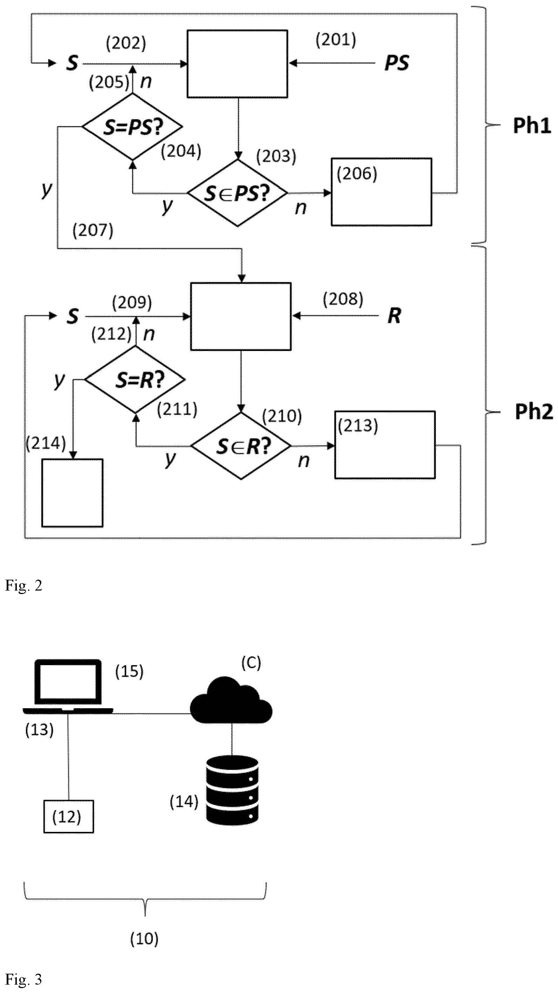

Fig. 2 zeigt schematisch und beispielhaft in Form eines Ablaufdiagramms die Schritte, die von einem Computer ausgeführt werden, in dessen Arbeitsspeicher das erfindungsgemäße Computerprogramm geladen ist. Die Schritte sind in zwei Phasen Ph1 und Ph2 unterteilt. Die Schritte (201), (202), (203), (204), (205) und (206) sind der ersten Phase zugeordnet. Die Schritte (208), (209), (210), (211), (212), (213) und (214) sind der zweiten Phase zugeordnet. Der Schritt (207) wird der ersten Phase zugordnet und führt zu einem Wechsel der ersten Phase Ph1 in die zweite Phase Ph2. Der Computer beginnt mit der Ausführung der Schritte in Phase Ph1 und wechselt bei Vorliegen definierter Bedingungen (siehe unten) in die Phase Ph2.

(201) Der Computer lädt mehrere Pathogen-Sequenzen *PS* aus einem Datenspeicher.

(202) Der Computer empfängt eine wachsende Sequenz S einer Nukleinsäure.

(203) Der Computer prüft, ob die die wachsende Sequenz S in den Pathogen-Sequenzen *PS* enthalten ist (*S*∈*PS*?).

(206) Für den Fall, dass die wachsende Sequenz S in keiner der Pathogen-Sequenzen *PS* enthalten ist, versendet der Computer ein Signal, das zum Abbruch der Sequenzierung der aktuell sequenzierten Nukleinsäure führt. Stattdessen wird eine neue Nukleinsäure sequenziert und der Computer empfängt eine wachsende Sequenz S der neuen Nukleinsäure.

(204) Ist eine wachsende Sequenz S in einer Pathogen-Sequenz *PS* enthalten, wird geprüft, ob anhand der vorliegenden Informationen davon ausgegangen werden kann, dass die Sequenz S einem spezifischen Pathogen aus *PS* zugeordnet werden kann *(S=PS?).*

(205) Für den Fall, dass die vorliegenden Informationen noch nicht ausreichen, um die Aussage zu treffen, dass die Sequenz von einem spezifischen Pathogen stammt, wird die Sequenzierung der Nukleinsäure fortgesetzt.

(207) Für den Fall, dass die vorliegenden Informationen ausreichen, um die Aussage zu treffen, dass die Sequenz von einem spezifischen Pathogen stammt, ist das Pathogen in der entsprechenden Probe identifiziert. Es kann eine Information über das Pathogen ausgegeben werden und der Computer versendet ein Signal, das zum Abbruch der Sequenzierung der aktuell sequenzierten Nukleinsäure führt. Ferner wird in die zweite Phase Ph2 der Sequenzierung gewechselt.

(208) Der Computer lädt mindestens eine resistenzanzeigende Sequenz R für das identifizierte Pathogen aus einem Datenspeicher, wobei die mindestens eine resistenzanzeigende Sequenz einen Resistenzmarker, eine resistenzfreie Sequenz und/oder eine Target-Sequenz umfassen kann.

(209) Der Computer empfängt eine neue wachsende Sequenz S einer Nukleinsäure.

(210) Der Computer prüft, ob die die wachsende Sequenz S in der mindestens einen resistenzanzeigenden Sequenz R enthalten ist (*S*∈*R*?).

(213) Für den Fall, dass die wachsende Sequenz *S* in keiner resistenzanzeigenden Sequenz enthalten ist, versendet der Computer ein Signal, das zum Abbruch der Sequenzierung der aktuell sequenzierten Nukleinsäure führt. Stattdessen wird eine neue Nukleinsäure sequenziert und der Computer empfängt eine wachsende Sequenz S der neuen Nukleinsäure.

(211) Ist die wachsende Sequenz S in der resistenzanzeigenden Sequenz R enthalten, wird geprüft, ob anhand der vorliegenden Informationen davon ausgegangen werden kann, dass das Pathogen über die entsprechende Resistenz verfügt (*S=R*?).

(212) Für den Fall, dass die vorliegenden Informationen noch nicht ausreichen, um die Aussage zu treffen, dass das Pathogen die Resistenz aufweist, wird die Sequenzierung der Nukleinsäure fortgesetzt.

(214) Für den Fall, dass die vorliegenden Informationen ausreichen, um die Aussage zu treffen, dass das Pathogen die vorliegende Resistenz aufweist, wird eine Information über die Resistenz ausgegeben.

Figur 3 zeigt schematisch eine weitere Ausführungsform des erfindungsgemäßen Systems. Das System (10) umfasst eine Sequenziereinheit (12), eine Steuer- und Berechnungseinheit (13), einen Datenspeicher (14) und eine Ausgabeeinheit (15).

Die Steuer- und Berechnungseinheit (13) und die Ausgabeeinheit (15) sind Bestandteile eines standmäßigen Computersystems, z.B. eines Laptops. Die Steuer- und Berechnungseinheit (13) empfängt Daten von der Sequenziereinheit (12), wobei die Daten wachsende Abfolgen von Nukleotiden oder Aminosäuren repräsentieren. Die Steuer- und Berechnungseinheit (13) ist ferner über ein Netzwerk (z.B. das Internet, hier repräsentiert durch eine Cloud (C)) mit der Datenbank (14) verbunden. Es ist denkbar, dass mehrere Datenbanken vorhanden sind. Es ist auch denkbar, dass eine Datenbank Bestandteil des Computersystems ist. Von der mindestens einen Datenbank bezieht die Steuer- und Berechnungseinheit (13) mindestens eine Pathogen-Sequenz und ggf. mindestens eine resistenzanzeigende Sequenz.

Es ist denkbar, dass die mindestens eine Datenbank auch Daten zu Umweltbedingungen, Daten zu Bekämpfungsmitteln, Daten zu Pflanzen und Pflanzensorten, Daten zu Pathogen und/oder dergleichen bereitstellen kann. Ferner ist denkbar, dass in der mindestens einen Datenbank ein Modell oder mehrere Modelle zur Berechnung der Wahrscheinlichkeit für das Auftreten eines Pathogens bei einer Pflanze zum aktuellen Zeitpunkt auf Basis von Informationen zu Pflanze und/oder zu den Umweltbedingungen gespeichert ist/sind, das/die von der Steuer- und Berechnungseinheit (13) abgerufen und für Berechnungszwecke genutzt werden kann/können.

## Patentansprüche

1. Verfahren umfassend die Schritte:
- Sammeln einer Probe von einer Pflanze (P) oder von einem Medium, in dem die Pflanze (P) wächst,
- Identifizieren eines Pathogens in der Probe,
- Identifizieren von Anzeichen auf eine Resistenz des Pathogens gegenüber einem Bekämpfungsmittel,
• wobei das Identifizieren des Pathogens und das Identifizieren der Anzeichen auf eine Resistenz mittels Sequenzieren von einzelnen Nukleinsäuren oder einzelnen Peptiden in der Probe erfolgt, wobei beim Sequenzieren der einzelnen Nukleinsäuren oder einzelnen Peptiden Sequenzen von Nukleotiden oder Sequenzen von Aminosäuren ermittelt werden, wobei die Sequenzen von Nukleotiden oder Sequenzen von Aminosäuren mit Referenzsequenzen verglichen werden,
**dadurch gekennzeichnet, dass** das Sequenzieren in zwei Phasen erfolgt, einer ersten Phase (Ph1) und einer zweiten Phase (Ph2),
• wobei in der ersten Phase (Ph1) während des Sequenzierens einer jeden Nukleinsäure oder eines jeden Peptids die jeweils ermittelte Sequenz (S) mit mindestens einer Pathogen-Sequenz (*PS*) verglichen wird, und
▪ für den Fall, dass die ermittelte Sequenz (S) nicht mit der mindestens einen Pathogen-Sequenz (PS) übereinstimmt, die weitere Sequenzierung dieser Nukleinsäure oder dieses Peptids abgebrochen wird,
▪ für den Fall, dass die ermittelte Sequenz (*S*) mit einer Pathogen-Sequenz (*PS*) übereinstimmt, ein Pathogen ermittelt wird, dass die Pathogen-Sequenz (PS) aufweist, und anhand des ermittelten Pathogens mindestens ein Resistenzmarker ermittelt wird, und in die zweite Phase (Ph2) gewechselt wird,
• wobei in der zweiten Phase (Ph2) Sequenzen der Nukleinsäuren oder Peptide in der Probe mit dem mindestens einen ermittelten Resistenzmarker und/oder mit einer resistenzfreien Sequenz verglichen werden, wobei eine resistenzfreie Sequenz eine Nukleotid-Abfolge oder eine Aminosäuren-Abfolge eines Pathogens ist, die keine Anzeichen auf eine Resistenz des Pathogens gegenüber einem Bekämpfungsmittel aufweist.

2. Verfahren gemäß Anspruch 1 umfassend die Schritte:
- Ermitteln einer Pflanze (P)
- Ermitteln von mindestens einem Pathogen, das die Pflanze (P) befallen kann
- Ermitteln von mindestens einer Pathogen-Sequenz (*PS*) für das mindestens eine ermittelte Pathogen
- Sammeln einer Probe von der Pflanze (P) oder von einem Medium, in dem die Pflanze (P) wächst
- Identifizieren eines Pathogens in der Probe
- Ermitteln von mindestens einem Resistenzmarker für das identifizierte Pathogen,
- Identifizieren von Anzeichen auf eine Resistenz des identifizierten Pathogens gegenüber einem Bekämpfungsmittel.

3. Verfahren gemäß Anspruch 1 umfassend die Schritte:
- Ermitteln einer Pflanze (P)
- Ermitteln von Umweltbedingungen, denen die Pflanze (P) ausgesetzt ist und/oder ausgesetzt gewesen ist
- Ermitteln von mindestens einem Pathogen, das die Pflanze (P) befallen kann
- Ermitteln von mindestens einer Pathogen-Sequenz (*PS*) für das mindestens eine ermittelte Pathogen
- Sammeln einer Probe von der Pflanze (P) oder von einem Medium, in dem die Pflanze (P) wächst
- Identifizieren eines Pathogens in der Probe
- Ermitteln von mindestens einem Resistenzmarker für das identifizierte Pathogen,
- Identifizieren von Anzeichen auf eine Resistenz des identifizierten Pathogens gegenüber einem Bekämpfungsmittel.

4. Verfahren gemäß Anspruch 1 umfassend die Schritte:
- Ermitteln einer Pflanze (P)
- Ermitteln von mindestens einem Pathogen, das die Pflanze (P) befallen kann
- Ermitteln von Umweltbedingungen, denen die Pflanze (P) ausgesetzt ist oder ausgesetzt gewesen ist,
- Berechnen einer Wahrscheinlichkeit für das Auftreten des mindestens einen Pathogens bei der Pflanze (P) zum aktuellen Zeitpunkt
- Vergleichen der Wahrscheinlichkeit mit einem vordefinierten Schwellenwert
- Ermitteln von Pathogen-Sequenzen (*PS*) für diejenigen ermittelten Pathogen, für die die Wahrscheinlichkeit oberhalb des Schwellenwerts liegt
- Sammeln einer Probe von der Pflanze (P) oder von einem Medium, in dem die Pflanze (P) wächst
- Identifizieren eines Pathogens in der Probe
- Ermitteln von mindestens einem Resistenzmarker für das identifizierte Pathogen,
- Identifizieren von Anzeichen auf eine Resistenz des identifizierten Pathogens gegenüber einem Bekämpfungsmittel.

5. Verfahren gemäß einem der Ansprüche 1 bis 4, wobei der Schritt Identifizieren von Anzeichen auf eine Resistenz des identifizierten Pathogens gegenüber einem Bekämpfungsmittel die folgenden Teilschritte umfasst:
- Vergleichen der Sequenzen von den Nukleinsäuren oder Peptiden in der Probe mit dem mindestens einen ermittelten Resistenzmarker,
- für den Fall, dass der Resistenzmarker in keiner der Sequenzen von den Nukleinsäuren oder Peptiden in der Probe auftritt: Ausgeben einer Mitteilung, dass für das identifizierte Pathogen kein Anzeichen einer Resistenz gefunden wurde,
- für den Fall, dass der Resistenzmarker in einer oder in mehreren der Sequenzen auftritt: Ausgeben einer Mitteilung, dass für das identifizierte Pathogen ein Anzeichen einer Resistenz gefunden wurde, und Ausgeben einer Mitteilung über die Resistenz.

6. Verfahren gemäß einem der Ansprüche 1 bis 4, wobei der Schritt Identifizieren von Anzeichen auf eine Resistenz des identifizierten Pathogens gegenüber einem Bekämpfungsmittel die folgenden Teilschritte umfasst:
- Vergleichen der Sequenzen von den Nukleinsäuren oder Peptiden in der Probe mit dem mindestens einen ermittelten Resistenzmarker,
- für den Fall, dass der Resistenzmarker in keiner der Sequenzen von den Nukleinsäuren oder Peptiden in der Probe auftritt oder in einer Anzahl von Nukleinsäuren auftritt, die kleiner als eine vordefinierte Mindestanzahl ist: Ausgeben einer Mitteilung, dass für das identifizierte Pathogen kein Anzeichen einer Resistenz gefunden wurde,
- für den Fall, dass der Resistenzmarker in einer Mindestanzahl der Sequenzen auftritt oder in einer Anzahl, die größer als die Mindestanzahl ist: Ausgeben einer Mitteilung, dass für das identifizierte Pathogen ein Anzeichen einer Resistenz gefunden wurde, und Ausgeben einer Mitteilung über die Resistenz.

7. Verfahren gemäß einem der Ansprüche 1 bis 6, umfassend die Schritte:
- Sammeln einer Probe von einer Pflanze (P) oder von einem Medium, in dem die Pflanze (P) wächst
- Identifizieren eines Pathogens in der Probe
- Ermitteln von mindestens einem Resistenzmarker für das identifizierte Pathogen und Ermitteln einer resistenzfreien Sequenz für das identifizierte Pathogen
- Identifizieren von Anzeichen auf eine Resistenz des identifizierten Pathogens gegenüber einem Bekämpfungsmittel durch Vergleichen der Sequenzen von Nukleinsäuren oder Peptiden in der Probe mit dem mindestens einen ermittelten Resistenzmarker und der resistenzfreien Sequenz
- für den Fall, dass eine Sequenz oder eine Anzahl an Sequenzen, die größer oder gleich einer Mindestanzahl ist, weder eine Übereinstimmung mit dem mindestens einen Resistenzmarker noch mit der resistenzfreien Sequenz aufweist: Ausgeben einer Mitteilung, dass in der Probe ein Pathogen identifiziert wurde, das eine Mutation aufweist, die auf eine neue und/oder sich ausbreitende Resistenz hindeutet.

8. Verfahren gemäß einem der Ansprüche 1 bis 6, umfassend die Schritte:
- Sammeln einer Probe von einer Pflanze (P) oder von einem Medium, in dem die Pflanze (P) wächst
- Identifizieren eines Pathogens in der Probe
- Ermitteln einer Target-Sequenz für das identifizierte Pathogen
- Identifizieren von Anzeichen auf eine neue Resistenz des identifizierten Pathogens gegenüber einem Bekämpfungsmittel durch Vergleichen der Sequenzen von Nukleinsäuren oder Peptiden in der Probe mit der Target-Sequenz
- für den Fall, dass eine Sequenz oder eine Anzahl an Sequenzen, die größer oder gleich einer Mindestanzahl ist, eine Mutation der Target-Sequenz darstellt: Ausgeben einer Mitteilung, dass in der Probe ein Pathogen identifiziert wurde, das eine Mutation aufweist, die auf eine neue und/oder sich ausbreitende Resistenz hindeutet.

9. Verfahren gemäß einem der Ansprüche 1 bis 6, umfassend die Schritte:
- Sammeln einer Probe von einer Pflanze (P) oder von einem Medium, in dem die Pflanze (P) wächst
- Identifizieren eines Pathogens in der Probe
- Ermitteln von mindestens einem Resistenzmarker für das identifizierte Pathogen und Ermitteln einer resistenzfreien Sequenz für das identifizierte Pathogen
- Vergleichen der Sequenzen von Nukleinsäuren oder Peptiden in der Probe mit dem mindestens einen ermittelten Resistenzmarker und der resistenzfreien Sequenz
- Ermitteln einer Häufigkeit *Q_{R}* von in der Probe gefundenen Nukleinsäuren oder Peptiden mit einer Sequenz gemäß dem Resistenzmarker, Ermitteln einer Häufigkeit *Q_{NR}* an in der Probe gefundenen Nukleinsäuren oder Peptiden mit einer Sequenz gemäß der resistenzfreien Sequenz, Berechnen eines Verhältnisses der ermittelten Häufigkeiten *Q_{R}* und *Q_{NR},* und vergleichen des Verhältnisses mit einem vordefinierten Mindestwert
- für den Fall, dass das Verhältnis größer oder gleich den Mindestwert ist: Ausgeben einer Mitteilung, dass für das identifizierte Pathogen ein Anzeichen einer Resistenz gefunden wurde, und Ausgeben einer Mitteilung über die Resistenz.

10. Verfahren gemäß einem der Ansprüche 1 bis 9, wobei es sich bei der Probe von der Pflanze (P) um ein Blatt oder mehrere Blätter von der Pflanze (P) handelt.

11. Verfahren gemäß einem der Ansprüche 1 bis 10, wobei sich die Pflanze (P) im Makrostadium 1 oder 2 oder 3 bis 6 gemäß BBCH-Skala befindet.

12. Verfahren gemäß einem der Ansprüche 1 bis 11, wobei es sich bei der Pathogen-Sequenz (PS) um eine DNS-Nukleotid-Abfolge von einem Pilz oder Bakterium, Virus handelt.

13. Verfahren gemäß einem der Ansprüche 1 bis 12, ferner umfassend die Schritte:
- Erstellen und/oder Aktualisieren einer Pathogenkarte, wobei in der Pathogenkarte ein Ort verzeichnet ist, wobei an dem Ort die Probe von der Pflanze (P) gesammelt worden ist, wobei für den Ort angegeben ist, ob in der Pflanze (P) ein Pathogen identifiziert worden ist und, für den Fall, dass in der Pflanze (P) ein Pathogen identifiziert worden ist, ob das Pathogen eine Resistenz gegenüber einem Bekämpfungsmittel aufweist, und
- Ausgeben der Pathogenkarte, vorzugsweise Anzeigen der Pathogenkarte auf einem Bildschirm und/oder Ausgeben der Pathogenkarte auf einem Drucker und/oder Übermitteln der Pathogenkarte an eine Vorrichtung zur automatisierten Ausführung einer Maßnahme zur Bekämpfung des identifizierten Pathogens.

14. Verfahren gemäß einem der Ansprüche 1 bis 13, ferner umfassend die Schritte:
- Ermitteln eines Bekämpfungsmittels zur Bekämpfung des identifizierten Pathogens unter Berücksichtigung der gegebenenfalls identifizierten Resistenz,
- Ausführen einer Maßnahme zur Bekämpfung des Pathogens bei der Pflanze unter Verwendung des ermittelten Bekämpfungsmittels.

15. System (10) umfassend
- eine Sequenziereinheit (12),
- eine Steuer- und Berechnungseinheit (13) und
- mindestens einen Datenspeicher (14), in dem für mindestens ein Pathogen mindestens eine Pathogen-Sequenz (PS) und mindestens ein Resistenzmarker gespeichert sind,
wobei die Sequenziereinheit (12) konfiguriert ist, Nukleinsäuren oder Peptide in einer Probe von einer Pflanze (P) oder von einem Medium, in dem die Pflanze (P) wächst, zu sequenzieren, und dabei Sequenzen von Nukleotiden oder Aminosäuren in der Probe zu ermitteln,
wobei die Sequenziereinheit (12) konfiguriert ist, während der Ermittlung der Sequenzen, die jeweils ermittelten Sequenzen an die Steuer- und Berechnungseinheit (13) zu übermitteln,
**dadurch gekennzeichnet, dass** die Steuer- und Berechnungseinheit (13) konfiguriert ist, in einer ersten Phase (Ph1) jede übermittelte Sequenz (S) von einer Nukleinsäure oder einem Peptid mit mindestens einer Pathogen-Sequenz (PS) zu vergleichen, und
• für den Fall, dass eine übermittelte Sequenz (*S*) nicht mit der mindestens einen Pathogen-Sequenz (*PS*) übereinstimmt, die Sequenziereinheit (12) zu veranlassen, die weitere Sequenzierung der jeweiligen Nukleinsäure oder des jeweiligen Peptids abzubrechen,
• für den Fall, dass eine übermittelte Sequenz (S) mit einer Pathogen-Sequenz (*PS*) übereinstimmt, ein Pathogen zu ermitteln, das die Pathogen-Sequenz (*PS*) aufweist, und anhand des ermittelten Pathogens mindestens einen Resistenzmarker zu ermitteln, und in eine zweite Phase (Ph2) zu wechseln,
wobei die Steuer- und Berechnungseinheit (13) konfiguriert ist, in der zweiten Phase (Ph2) Sequenzen der Nukleinsäuren oder Peptide in der Probe mit dem mindestens einen ermittelten Resistenzmarker und/oder einer resistenzfreien Sequenz zu vergleichen, wobei eine resistenzfreie Sequenz eine Nukleotid-Abfolge oder eine Aminosäuren-Abfolge eines Pathogens ist, die keine Anzeichen auf eine Resistenz des Pathogens gegenüber einem Bekämpfungsmittel aufweist.

16. Computerlesbares (Speicher-)Medium umfassend Befehle, die bei einer Ausführung durch einen Computer diesen veranlassen, folgenden Schritte auszuführen:
- für eine Vielzahl von Nukleinsäuren oder Peptiden: Empfangen einer wachsenden Abfolge von Nukleotiden oder Aminosäuren in der jeweiligen Nukleinsäure oder dem jeweiligen Peptid von einer Sequenziereinheit (12),
**dadurch gekennzeichnet, dass** die Befehle den Computer ferner veranlassen, folgende Schritte auszuführen:
- in einer ersten Phase (Ph1) während des Empfangens der wachsenden Abfolge: Prüfen, ob die Abfolge von Nukleotiden oder Aminosäuren ein- oder mehrfach in mindestens einer Pathogen-Sequenz (PS) enthalten ist,
- für den Fall, dass die Abfolge in keiner Pathogen-Sequenz (*PS*) enthalten ist: Übermitteln eines Signals an die Sequenziereinheit (12) zum Abbruch der weiteren Sequenzierung der Nukleinsäure oder des Peptids,
- für den Fall, dass die Abfolge mit einer Pathogen-Sequenz (*PS*) übereinstimmt: Ermitteln eines Pathogens, das die Pathogen-Sequenz aufweist, und Ermitteln mindestens eines Referenzmarkers, und Initiieren einer zweiten Phase (Ph2),
- in der zweiten Phase (Ph2): Vergleichen der Abfolgen von Nukleotiden oder Aminosäuren mit dem mindestens einen Referenzmarker und/oder einer resistenzfreien Sequenz, wobei eine resistenzfreie Sequenz eine Nukleotid-Abfolge oder eine Aminosäuren-Abfolge eines Pathogens ist, die keine Anzeichen auf eine Resistenz des Pathogens gegenüber einem Bekämpfungsmittel aufweist.

## Claims

1. Method comprising the steps of:
- collecting a sample of a plant (P) or of a medium in which the plant (P) is growing,
- identifying a pathogen in the sample,
- identifying signs of a resistance of the pathogen to a control agent,
• wherein the identification of the pathogen and the identification of the signs of a resistance is done by means of sequencing of individual nucleic acids or individual peptides in the sample, wherein sequences of nucleotides or sequences of amino acids are ascertained in the sequencing of the individual nucleic acids or individual peptides, wherein the sequences of nucleotides or sequences of amino acids are compared with reference sequences,
**characterized in that** the sequencing is done in two phases, a first phase (Ph1) and a second phase (Ph2),
• wherein, in the first phase (Ph1), during the sequencing of each nucleic acid or each peptide, the respectively ascertained sequence (S) is compared with at least one pathogen sequence (*PS*), and
▪ if the ascertained sequence (*S*) does not match the at least one pathogen sequence (*PS*), further sequencing of said nucleic acid or said peptide is aborted,
• if the ascertained sequence (*S*) matches a pathogen sequence *(PS),* a pathogen having the pathogen sequence *(PS)* is ascertained and the ascertained pathogen is used to ascertain at least one resistance marker, and a switch is made to the second phase (Ph2),
• wherein, in the second phase (Ph2), sequences of the nucleic acids or peptides in the sample are compared with the at least one ascertained resistance marker and/or with a resistance-free sequence, wherein a resistance-free sequence is a succession of nucleotides or a succession of amino acids of a pathogen that does not exhibit signs of a resistance of the pathogen to a control agent.

2. Method according to Claim 1, comprising the steps of:
- ascertaining a plant (P)
- ascertaining at least one pathogen which can attack the plant (P)
- ascertaining at least one pathogen sequence (PS) for the at least one ascertained pathogen
- collecting a sample of the plant (P) or of a medium in which the plant (P) is growing
- identifying a pathogen in the sample
- ascertaining at least one resistance marker for the identified pathogen,
- identifying signs of a resistance of the identified pathogen to a control agent.

3. Method according to Claim 1, comprising the steps of:
- ascertaining a plant (P)
- ascertaining environmental conditions to which the plant (P) is exposed and/or has been exposed
- ascertaining at least one pathogen which can attack the plant (P)
- ascertaining at least one pathogen sequence (PS) for the at least one ascertained pathogen
- collecting a sample of the plant (P) or of a medium in which the plant (P) is growing
- identifying a pathogen in the sample
- ascertaining at least one resistance marker for the identified pathogen,
- identifying signs of a resistance of the identified pathogen to a control agent.

4. Method according to Claim 1, comprising the steps of:
- ascertaining a plant (P)
- ascertaining at least one pathogen which can attack the plant (P)
- ascertaining environmental conditions to which the plant (P) is exposed or has been exposed,
- calculating a probability of the at least one pathogen occurring currently for the plant (P)
- comparing the probability with a predefined threshold value
- ascertaining pathogen sequences (PS) for those ascertained pathogens for which the probability is above the threshold value
- collecting a sample of the plant (P) or of a medium in which the plant (P) is growing
- identifying a pathogen in the sample
- ascertaining at least one resistance marker for the identified pathogen,
- identifying signs of a resistance of the identified pathogen to a control agent.

5. Method according to any of Claims 1 to 4, wherein the step of identifying signs of a resistance of the identified pathogen to a control agent comprises the following partial steps:
- comparing the sequences of the nucleic acids or peptides in the sample with the at least one ascertained resistance marker,
- if the resistance marker does not occur in any of the sequences of the nucleic acids or peptides in the sample: outputting a notification indicating that no sign of a resistance was found for the identified pathogen,
- if the resistance marker occurs in one or more of the sequences: outputting a notification indicating that a sign of a resistance was found for the identified pathogen, and outputting a notification about the resistance.

6. Method according to any of Claims 1 to 4, wherein the step of identifying signs of a resistance of the identified pathogen to a control agent comprises the following partial steps:
- comparing the sequences of the nucleic acids or peptides in the sample with the at least one ascertained resistance marker,
- if the resistance marker does not occur in any of the sequences of the nucleic acids or peptides in the sample or occurs in a number of nucleic acids that is smaller than a predefined minimum number: outputting a notification indicating that no sign of a resistance was found for the identified pathogen,
- if the resistance marker occurs in a minimum number of sequences or in a number greater than the minimum number: outputting a notification indicating that a sign of a resistance was found for the identified pathogen, and outputting a notification about the resistance.

7. Method according to any of Claims 1 to 6, comprising the steps of:
- collecting a sample of a plant (P) or of a medium in which the plant (P) is growing
- identifying a pathogen in the sample
- ascertaining at least one resistance marker for the identified pathogen and ascertaining a resistance-free sequence for the identified pathogen
- identifying signs of a resistance of the identified pathogen to a control agent by comparison of the sequences of nucleic acids or peptides in the sample with the at least one ascertained resistance marker and the resistance-free sequence
- if a sequence or a number of sequences that is greater than or equal to a minimum number does not have a match with either the at least one resistance marker or the resistance-free sequence: outputting a notification indicating that a pathogen having a mutation pointing to a new and/or spreading resistance was identified in the sample.

8. Method according to any of Claims 1 to 6, comprising the steps of:
- collecting a sample of a plant (P) or of a medium in which the plant (P) is growing
- identifying a pathogen in the sample
- ascertaining a target sequence for the identified pathogen
- identifying signs of a new resistance of the identified pathogen to a control agent by comparison of the sequences of nucleic acids or peptides in the sample with the target sequence
- if a sequence or a number of sequences that is greater than or equal to a minimum number represents a mutation of the target sequence: outputting a notification indicating that a pathogen having a mutation pointing to a new and/or spreading resistance was identified in the sample.

9. Method according to any of Claims 1 to 6, comprising the steps of:
- collecting a sample of a plant (P) or of a medium in which the plant (P) is growing
- identifying a pathogen in the sample
- ascertaining at least one resistance marker for the identified pathogen and ascertaining a resistance-free sequence for the identified pathogen
- comparing the sequences of nucleic acids or peptides in the sample with the at least one ascertained resistance marker and the resistance-free sequence
- ascertaining a frequency *Q_{R}* of nucleic acids or peptides found in the sample that have a sequence according to the resistance marker, ascertaining a frequency *Q_{NR}* of nucleic acids or peptides found in the sample that have a sequence according to the resistance-free sequence, calculating a ratio of the ascertained frequencies *Q_{R}* and *Q_{NR},* and comparing the ratio with a predefined minimum value
- if the ratio is greater than or equal to the minimum value: outputting a notification indicating that a sign of a resistance was found for the identified pathogen, and outputting a notification about the resistance.

10. Method according to any of Claims 1 to 9, wherein the sample of the plant (P) is a leaf or multiple leaves of the plant (P).

11. Method according to any of Claims 1 to 10, wherein the plant (P) is in principal growth stage 1 or 2 or 3 to 6 according to the BBCH scale.

12. Method according to any of Claims 1 to 11, wherein the pathogen sequence *(PS)* is a succession of DNA nucleotides from a fungus or bacterium or virus.

13. Method according to any of Claims 1 to 12, further comprising the steps of:
- creating and/or updating a pathogen map, wherein a location is recorded in the pathogen map, wherein the sample of the plant (P) has been collected at the location, wherein an indication is given for the location as to whether a pathogen has been identified in the plant (P) and, if a pathogen has been identified in the plant (P), whether the pathogen has a resistance to a control agent, and
- outputting the pathogen map, preferably displaying the pathogen map on a screen and/or outputting the pathogen map on a printer and/or transmitting the pathogen map to a device for automatic execution of a measure to control the identified pathogen.

14. Method according to any of Claims 1 to 13, further comprising the steps of:
- ascertaining a control agent for controlling the identified pathogen, taking into account the resistance that may have been identified,
- carrying out a measure to control the pathogen in the plant using the ascertained control agent.

15. System (10) comprising
- a sequencing unit (12),
- a control and calculation unit (13) and
- at least one data store (14) in which at least one pathogen sequence (PS) and at least one resistance marker are stored for at least one pathogen,
wherein the sequencing unit (12) is configured to sequence nucleic acids or peptides in a sample of a plant (P) or of a medium in which the plant (P) is growing and to thereby ascertain sequences of nucleotides or amino acids in the sample,
wherein the sequencing unit (12) is configured to transmit, during the ascertainment of the sequences, the respectively ascertained sequences to the control and calculation unit (13),
**characterized in that** the control and calculation unit (13) is configured to compare, in a first phase (Ph1), each transmitted sequence (*S*) of a nucleic acid or a peptide with at least one pathogen sequence (*PS*), and
• if a transmitted sequence (S) does not match the at least one pathogen sequence *(PS)*, to prompt the sequencing unit (12) to abort further sequencing of the respective nucleic acid or the respective peptide,
• if a transmitted sequence (*S*) matches a pathogen sequence (*PS*), to ascertain a pathogen having the pathogen sequence (*PS*) and to use the ascertained pathogen to ascertain at least one resistance marker, and to make a switch to a second phase (Ph2),
wherein the control and calculation unit (13) is configured to compare, in the second phase (Ph2), sequences of the nucleic acids or peptides in the sample with the at least one ascertained resistance marker and/or a resistance-free sequence, wherein a resistance-free sequence is a succession of nucleotides or a succession of amino acids of a pathogen that does not exhibit signs of a resistance of the pathogen to a control agent.

16. Computer-readable (storage) medium comprising commands which, upon execution by a computer, prompt said computer to execute the following steps:
- for a multiplicity of nucleic acids or peptides: receiving a growing succession of nucleotides or amino acids in the respective nucleic acid or the respective peptide from a sequencing unit (12),
**characterized in that** the commands also prompt the computer to execute the following steps:
- in a first phase (Ph1) while the growing succession is being received: checking whether the succession of nucleotides or amino acids is singly or multiply present in at least one pathogen sequence (*PS*),
- if the succession is not present in any pathogen sequence (*PS*): transmitting a signal to the sequencing unit (12) to abort further sequencing of the nucleic acid or the peptide,
- if the succession matches a pathogen sequence (*PS*): ascertaining a pathogen having the pathogen sequence, and ascertaining at least one reference marker, and initiating a second phase (Ph2),
- in the second phase (Ph2): comparing the successions of nucleotides or amino acids with the at least one reference marker and/or a resistance-free sequence, wherein a resistance-free sequence is a succession of nucleotides or a succession of amino acids of a pathogen that does not exhibit signs of a resistance of the pathogen to a control agent.

## Revendications

1. Procédé comprenant les étapes :
- collecte d'un échantillon d'une plante (P) ou d'un milieu dans lequel pousse la plante (P),
- identification d'un agent pathogène dans l'échantillon,
- identification de signes d'une résistance de l'agent pathogène à un agent de lutte,
• l'identification de l'agent pathogène et l'identification des signes d'une résistance s'effectuant au moyen de séquences d'acides nucléiques individuels ou de peptides individuels dans l'échantillon, lors du séquençage des acides nucléiques individuels ou peptides individuels des séquences de nucléotides ou des séquences d'acides aminés étant déterminées, les séquences de nucléotides ou séquences d'acides aminés étant comparées à des séquences de référence,
**caractérisé en ce que** le séquençage s'effectue en deux phases, une première phase (Ph1) et une seconde phase (Ph2),
• dans la première phase (Ph1) pendant le séquençage d'un tel acide nucléique ou d'un tel peptide la séquence déterminée (*S*) dans chaque cas est comparée à au moins une séquence d'agent pathogène (*PS*), et
▪ dans le cas où la séquence déterminée (*S*) ne coïncide pas avec ladite au moins une séquence d'agent pathogène *(PS),* la poursuite du séquençage de cet acide nucléique ou de ce peptide est interrompue,
▪ dans le cas où la séquence déterminée (S) coïncide avec une séquence d'agent pathogène (PS), un agent pathogène qui comporte la séquence d'agent pathogène (PS) est déterminé, et à l'aide de l'agent pathogène déterminé on détermine au moins un marqueur de résistance, et on passe à la seconde phase (Ph2),
• dans la seconde phase (Ph2) des séquences des acides nucléiques ou peptides dans l'échantillon étant comparées audit au moins un marqueur de résistance déterminé et/ou à une séquence exempte de résistance, une séquence exempte de résistance étant une suite de nucléotides ou une suite d'acides aminés d'un agent pathogène, qui ne présente aucun signe d'une résistance de l'agent pathogène à un agent de lutte.

2. Procédé selon la revendication 1, comprenant les étapes :
- détermination d'une plante (P)
- détermination d'au moins un agent pathogène qui peut attaquer la plante (P)
- détermination d'au moins une séquence d'agent pathogène (PS) dudit au moins un agent pathogène déterminé
- collecte d'un échantillon de la plante (P) ou d'un milieu dans lequel pousse la plante (P),
- identification d'un agent pathogène dans l'échantillon
- détermination d'au moins un marqueur de résistance pour l'agent pathogène identifié,
- identification de signes d'une résistance de l'agent pathogène à un agent de lutte,

3. Procédé selon la revendication 1, comprenant les étapes :
- détermination d'une plante (P)
- détermination de conditions environnementales auxquelles la plante (P) est exposée et/ou a été exposée
- détermination d'au moins un agent pathogène qui peut attaquer la plante (P)
- détermination d'au moins une séquence d'agent pathogène (PS) dudit au moins un agent pathogène déterminé
- collecte d'un échantillon de la plante (P) ou d'un milieu dans lequel pousse la plante (P),
- identification d'un agent pathogène dans l'échantillon,
- détermination d'au moins un marqueur de résistance pour l'agent pathogène identifié,
- identification de signes d'une résistance de l'agent pathogène à un agent de lutte,

4. Procédé selon la revendication 1, comprenant les étapes :
- détermination d'une plante (P)
- détermination d'au moins un agent pathogène qui peut attaquer la plante (P)
- détermination de conditions environnementales auxquelles la plante (P) est exposée et/ou a été exposée
- calcul d'une probabilité de l'apparition dudit au moins un agent pathogène chez la plante (P) à l'instant actuel
- comparaison de la probabilité avec une valeur seuil définie
- détermination de séquences d'agents pathogènes (PS) des agents pathogènes déterminés pour lesquels la probabilité est supérieure à la valeur seuil
- collecte d'un échantillon de la plante (P) ou d'un milieu dans lequel pousse la plante (P),
- identification d'un agent pathogène dans l'échantillon,
- détermination d'au moins un marqueur de résistance pour l'agent pathogène identifié,
- identification de signes d'une résistance de l'agent pathogène à un agent de lutte,

5. Procédé selon l'une quelconque des revendications 1 à 4, dan lequel l'étape d'identification de signes d'une résistance de l'agent pathogène identifié à un agent de lutte comprend les étapes partielles suivantes :
- comparaison des séquences des acides nucléiques ou peptides dans l'échantillon avec ledit au moins un marqueur de résistance déterminé,
- dans le cas où le marqueur de résistance n'apparaît dans aucune des séquences des acides nucléiques ou peptides dans l'échantillon : émission d'un message indiquant qu'aucun signe d'une résistance n'a été trouvé pour l'agent pathogène identifié,
- dans le cas où le marqueur de résistance apparaît dans une ou plusieurs des séquences : émission d'un message indiquant qu'un signe d'une résistance a été trouvé pour l'agent pathogène identifié, et émission d'un message relatif à la résistance.

6. Procédé selon l'une quelconque des revendications 1 à 4, dan lequel l'étape d'identification de signes d'une résistance de l'agent pathogène identifié à un agent de lutte comprend les étapes partielles suivantes :
- comparaison des séquences des acides nucléiques ou peptides dans l'échantillon avec ledit au moins un marqueur de résistance déterminé,
- dans le cas où le marqueur de résistance n'apparaît dans aucune des séquences des acides nucléiques ou peptides dans l'échantillon ou apparaît dans un nombre d'acides nucléiques qui est inférieur à un nombre minimum prédéfini : émission d'un message indiquant qu'aucun signe d'une résistance n'a été trouvé pour l'agent pathogène identifié,
- dans le cas où le marqueur de résistance apparaît dans un nombre minimum des séquences ou dans un nombre qui est supérieur au nombre minimum : émission d'un message indiquant qu'un signe d'une résistance a été trouvé pour l'agent pathogène identifié, et émission d'un message relatif à la résistance.

7. Procédé selon l'une quelconque des revendications 1 à 6, comprenant les étapes :
- collecte d'un échantillon d'une plante (P) ou d'un milieu dans lequel pousse la plante (P),
- identification d'un agent pathogène dans l'échantillon
- détermination d'au moins un marqueur de résistance pour l'agent pathogène identifié et détermination d'une séquence exempte de résistance pour l'agent pathogène identifié
- identification de signes d'une résistance de l'agent pathogène identifié à un agent de lutte par comparaison des séquences d'acides nucléiques ou de peptides dans l'échantillon avec ledit au moins un marqueur de résistance déterminé et la séquence exempte de résistance
- dans le cas où une séquence ou un nombre de séquences qui est supérieur ou égal à un nombre minimum ne coïncide ni avec ledit au moins un marqueur de résistance ni avec la séquence exempte de résistance : émission d'un message indiquant que dans l'échantillon a été identifié un agent pathogène qui comporte une mutation indicatrice d'une résistance nouvelle et/ou en extension.

8. Procédé selon l'une quelconque des revendications 1 à 6**,** comprenant les étapes :
- collecte d'un échantillon d'une plante (P) ou d'un milieu dans lequel pousse la plante (P),
- identification d'un agent pathogène dans l'échantillon,
- détermination d'une séquence cible pour l'agent pathogène identifié
- identification de signes d'une nouvelle résistance de l'agent pathogène identifié à un agent de lutte par comparaison des séquences d'acides nucléiques ou de peptides dans l'échantillon avec la séquence cible
- dans le cas où une séquence ou un nombre de séquences qui est supérieur ou égal à un nombre minimum représente une mutation de la séquence cible : émission d'un message indiquant que dans l'échantillon a été identifié un agent pathogène qui comporte une mutation indicatrice d'une résistance nouvelle et/ou en extension.

9. Procédé selon l'une quelconque des revendications 1 à 6, comprenant les étapes :
- collecte d'un échantillon d'une plante (P) ou d'un milieu dans lequel pousse la plante (P),
- identification d'un agent pathogène dans l'échantillon,
- détermination d'au moins un marqueur de résistance pour l'agent pathogène identifié et détermination d'une séquence exempte de résistance pour l'agent pathogène identifié
- comparaison des séquences d'acides nucléiques ou de peptides dans l'échantillon avec ledit au moins un marqueur de résistance déterminé et la séquence exempte de résistance
- détermination d'une fréquence Q_{R} d'acides nucléiques ou de peptides, trouvés dans l'échantillon, comportant une séquence conforme au marqueur de résistance, détermination d'une fréquence Q_{NR} d'acides nucléiques ou de peptides trouvés dans l'échantillon qui comportent une séquence conforme à la séquence exempte de résistance, calcul d'un rapport des fréquences déterminées Q_{R} et Q_{NR} et comparaison du rapport avec une valeur minimale prédéfinie
- dans le cas où le rapport est supérieur ou égal à la valeur minimale ; émission d'un message indiquant qu'un signe d'une résistance a été trouvé pour l'agent pathogène identifié, et émission d'un message relatif à la résistance.

10. Procédé selon l'une quelconque des revendications 1 à 9, dans lequel pour ce qui est de l'échantillon de la plante (P) il s'agit d'une feuille ou de plusieurs feuilles de la plante (P).

11. Procédé selon l'une quelconque des revendications 1 à 10, dans lequel la plante (P) se trouve dans le stade principal 1 ou 2 ou 3 à 6 selon l'échelle BBCH.

12. Procédé selon l'une quelconque des revendications 1 à 11, dans lequel pour ce qui est de la séquence d'agent pathogène (PS) il s'agit d'une suite ne nucléotides d'ADN d'un champignon, d'une bactérie ou d'un virus.

13. Procédé selon l'une quelconque des revendications 1 à 12, comprenant en outre les étapes :
- élaboration et/ou actualisation d'une carte d'agent pathogène, dans la carte d'agent pathogène étant indiqué un site, l'échantillon de la plante (P) ayant été récolté dans le site, pour le site étant indiqué si un agent pathogène a été identifié dans la plante (P) et, dans le cas où un agent pathogène a été identifié dans la plante (P), si l'agent pathogène présente une résistance à un agent de lutte, et
- émission de la carte d'agent pathogène, de préférence affichage de la carte d'agent pathogène sur un écran et/ou sortie de la carte d'agent pathogène sur une imprimante et/ou transmission de la carte d'agent pathogène à l'exécution automatisée d'une disposition destinée à la lutte contre l'agent pathogène identifié.

14. Procédé selon l'une quelconque des revendications 1 à 13, comprenant en outre les étapes :
- détermination d'un agent de lutte destiné à la lutte contre l'agent pathogène identifié avec prise en considération de la résistance éventuellement identifiée,
- exécution d'une disposition destinée à la lutte contre l'agent pathogène chez la plante avec utilisation de l'agent de lutte déterminé.

15. Système (10) comprenant
- une unité de séquençage (12),
- une unité de commande et de calcul (13), et
- au moins une mémoire de données (14), dans laquelle au moins une séquence d'agent pathogène (PS) et au moins un marqueur de résistance sont mémorisés pour au moins un agent pathogène,
l'unité de séquençage (12) étant configurée pour séquencer des acides nucléiques ou des peptides dans un échantillon d'une plante (P) ou d'un milieu dans lequel pousse la plante (P), et pour ainsi déterminer des séquences de nucléotides ou d'acides aminés dans l'échantillon.
l'unité de séquençage (12) étant configurée pour transmettre à l'unité de commande et de calcul (13), pendant la détermination des séquences, les séquences chaque fois déterminées,
**caractérisé en ce que** l'unité de commande et de calcul (13) est configurée pour, dans une première phase (Ph1), comparer chaque séquence déterminée (S) d'un acide nucléique ou d'un peptide avec au moins une séquence d'agent pathogène (PS), et
• dans le cas où une séquence (S) transmise ne coïncide pas avec ladite au moins une séquence d'agent pathogène (PS), amener l'unité de séquençage (12) à interrompre la poursuite du séquençage de l'acide aminé respectif ou du peptide respectif,
• dans le cas où la séquence déterminée (S) coïncide avec une séquence d'agent pathogène (PS), déterminer un agent pathogène qui comporte la séquence d'agent pathogène (PS), et déterminer à l'aide de l'agent pathogène déterminé au moins un marqueur de résistance, et passer dans une seconde phase (Ph2),
l'unité de commande et de calcul (13) étant configurée pour, dans la seconde phase (Ph2), comparer des séquences des acides nucléiques ou peptides dans l'échantillon audit au moins un marqueur de résistance déterminé et/ou à une séquence exempte de résistance, une séquence exempte de résistance étant une suite de nucléotides ou une suite d'acides aminés d'un agent pathogène, qui ne présente aucun signe d'une résistance de l'agent pathogène à un agent de lutte.

16. Support (de mémoire) lisible par ordinateur, comprenant des instructions qui, lors d'une exécution par un ordinateur, amènent ce dernier à exécuter les étapes suivantes :
- pour une pluralité d'acides nucléiques ou de peptides : capture d'une suite croissante de nucléotides ou d'acides aminés dans l'acide nucléique respectif ou le peptide respectif d'une unité de séquençage (12), **caractérisé en ce que** les instructions amènent en outre l'ordinateur à exécuter les étapes suivantes
- dans une première phase (Ph1) pendant la capture de la suite croissante : vérifier si la suite de nucléotides ou d'acides aminés est contenue une ou plusieurs fois dans au moins une séquence d'agent pathogène (PS),
- dans le cas où la suite n'est contenue dans aucune séquence d'agent pathogène (PS) : transmettre à l'unité de séquençage (12) un signal visant à l'interruption de la poursuite du séquençage de l'acide nucléique ou du peptide,
- dans le cas où la suite coïncide avec une séquence d'agent pathogène (PS) : déterminer un agent pathogène qui comporte la séquence d'agent pathogène, et déterminer au moins un marqueur de référence, et commencer une seconde phase (Ph2),
- dans la seconde phase (Ph2) : comparer la suite de nucléotides ou d'acides aminés avec ledit au moins un marqueur de référence et/ou une séquence exempte de résistance, une séquence exempte de résistance étant une suite de nucléotides ou une suite d'acides aminés d'un agent pathogène, qui ne présente aucun signe d'une résistance de l'agent pathogène à un agent de lutte.
